# EUROPEAN PATENT SPECIFICATION

(11) **EP 3 092 994 B1**
(45) Date of publication and mention of the grant of the patent: **06.02.2019**
(21) Application number: 14877587.7
(22) Date of filing: 09.10.2014
(51) Int. Cl.: B29C 65/08, A61F 13/15, A61F 13/49, B29C 65/78, B29L 31/48

(54) **ULTRASONIC WELDING APPARATUS AND ULTRASONIC WELDING METHOD OF SHEET-LIKE MEMBER ASSOCIATED WITH ABSORBENT ARTICLE**
ULTRASCHALLSCHWEISSVORRICHTUNG UND ULTRASCHALLSCHWEISSVERFAHREN FÜR BAHNENFÖRMIGE WERKSTÜCKE IN VERBINDUNG MIT SAUGFÄHIGEN ARTIKELN
DISPOSITIF DE SOUDAGE PAR ULTRASONS ET PROCÉDÉ DE SOUDAGE PAR ULTRASONS D'UN ÉLÉMENT EN FORME DE FEUILLE ASSOCIÉ À UN ARTICLE ABSORBANT

(30) Priority: 10.01.2014 JP 2014002994
(43) Date of publication of application: 16.11.2016
(73) Proprietor: Unicharm Corporation, Ehime 799-0111 (JP)
(72) Inventor: YAMAMOTO, Hiroki, Kanonji-shi Kagawa 769-1602 (JP); MATSUMOTO, Yoshihiko, Kanonji-shi Kagawa 769-1602 (JP)
(74) Representative: Dolleymores
(86) International application number: PCT/JP2014/077105
(87) International publication number: WO 2015/104881

(56) References cited:
- WO-A1-2012/042842
- WO-A1-2013/141022
- CN-A- 1 179 128
- JP-A- H0 857 959
- JP-A- H10 291 082
- JP-A- H10 513 128
- JP-A- 2000 015 701
- JP-A- 2010 094 883
- JP-A- 2013 233 729
- US-A- 5 643 396
- US-A- 5 667 608

## Description

### Technical Field

The invention relates to an ultrasonic welding apparatus and an ultrasonic welding method to weld a sheet-like member using ultrasonic vibration, the sheet-like member being associated with an absorbent article such as a disposable diaper.

### Background Art

In a conventional production line of an absorbent article such as a disposable diaper, a sheet-like member 1a, which is made of a plurality of stacked continuous sheets (e.g. nonwoven fabric), is welded so that these continuous sheets are joined. As an example of an apparatus 120 which performs such a welding process, Patent Literature 1 discloses an ultrasonic welding apparatus 120. The apparatus 120 generates friction heat by applying ultrasonic energy to the sheet-like member 1a, and thereby welds the sheet-like member 1a.

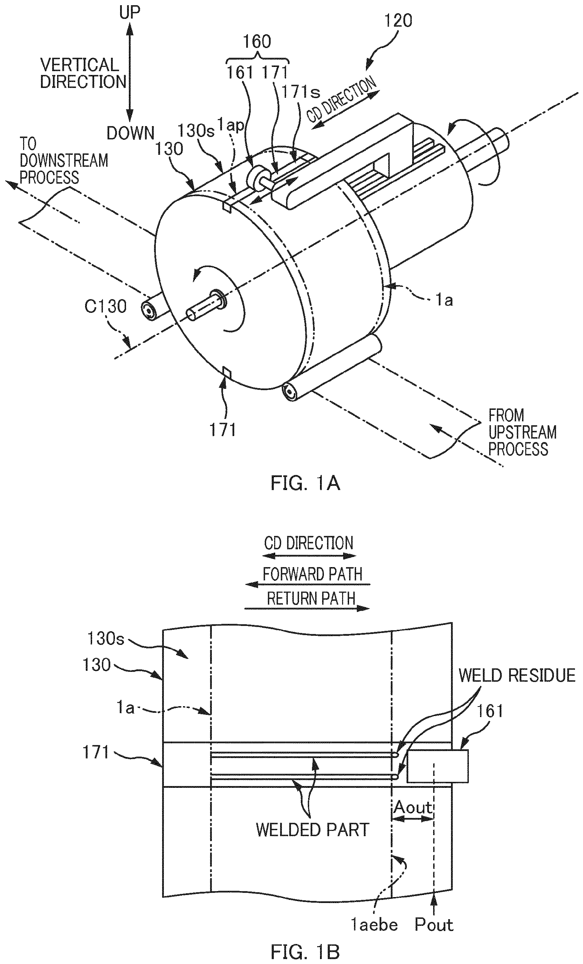

Fig. 1A is a schematic perspective view of the apparatus 120. The apparatus 120 includes a rotating drum 130 which rotates about a central axis C130. A sheet-like member 1a to be welded (see double-dotted chain lines in Fig. 1A) is wound around an outer circumferential face 130s of the rotating drum 130, and the rotating drum 130 rotates about the central axis C130. Accordingly, the sheet-like member 1a is substantially integrated with the outer circumferential face 130s of the rotating drum 130 and is transported.

The apparatus 120 also includes an ultrasonic processing unit 160, and the unit 160 rotates about the central axis C130 in an integrated manner with the rotating drum 130. During the rotation, the unit 160 performs ultrasonic welding process to the sheet-like member 1a.

That is, the ultrasonic processing unit 160 includes a rail-like anvil 171 and a roller-like horn 161 on the outer circumferential face 130s of the rotating drum 130. The anvil 171 extends in the CD direction, which is orthogonal to the transport direction. The horn 161 vibrates ultrasonically and is provided outside with respect to the anvil 171 in the rotation-radius direction of the rotating drum 130. During a period in which the horn 161 and the anvil 171 both are rotating about the central axis C130 together with the rotating drum 130 and the sheet-like member 1a, the roller-like horn 161 rolls on the anvil 171 along the CD direction and reciprocates in the CD direction. At this time, the horn 161 applies ultrasonic energy to a part 1a of the sheet-like member 1a which is placed on the anvil 171, and thereby welds the part 1ap.

### Citation List

### [Patent Literature].

[Patent Literature 1] Japanese Unexamined Patent Application Publication (Translation of PCT Application) No. 10-513128. US 5667608 A and EP0807013 A1 are patent family members.
WO 2013/141022 A1, WO 2012/042842 A1, CN 1179128 A and US 5643396 A also relate to ultrasonic processing systems for webs.

### Summary

### Technical Problem

In PTL 1, there is described that when the horn 161 that has reciprocated along the CD direction to perform ultrasonic welding has reached a crossed-over position Pout (Fig. 1B) that is located beyond the sheet-like member 1a, in other words a position Pout where there is no sheet-like member 1a, the horn 161 is held up from the anvil 171. Thus, with the ultrasonic welding apparatus 120 in PTL 1, ultrasonic energy is applied to the end edge 1aebe in the CD direction of the sheet-like member 1a.

In this case however, there is a possibility that weld residue may jut out from the end edge 1aebe of the sheet-like member 1a and remain thereon. Fig. 1B is an explanatory view of the above, and shows the sheet-like member 1a in a wrapped state on the outer circumferential face 130s of the rotating drum 130.

As shown in Fig. 1B, first, the horn 161 moves in the CD direction during ultrasonic welding, thus the melt that has melted from the sheet-like member 1a according to the welding process tends to be pushed out to a downstream side in the traveling direction of the horn 161 that moves in the CD direction. Thus, in the case where ultrasonic energy of a size necessary for welding is applied to a crossed-over position Pout that is located beyond the sheet-like member 1a in a return path as described above, at the time of finishing crossing of the end edge 1aebe of the sheet-like member 1a in the CD direction, the melt may jut out from the end edge 1aebe of the sheet-like member 1a in the CD direction. When the above melt hardens, the weld residues that have jutted out as burr will remain on the end edge 1aebe, making the appearance of the diaper unattractive.

The horn 161 is held up from the anvil 171 at the crossed-over position Pout that is located beyond the sheet-like member 1a in the CD direction, thus in a region Aout between the end edge 1aebe of the sheet-like member 1a and the crossed-over position Pout that is located beyond the end edge 1aebe to the outside in the CD direction, the horn 161 and the anvil 171 are in a contacting state. Then, the anvil 171 is directly hit with the horn 161 that ultrasonically vibrates to apply ultrasonic energy. Thus, there is a possibility that the horn 161 and the anvil 171 may wear or may break, or generated abrasion powder may attach to the sheet-like member 1a.

The present invention has been made in view of the above problems, and a purpose of the invention is to prevent weld residue from remaining on an end edge in a CD direction of a sheet-like member, and further to prevent a malfunction such as wear and breakage that may occur when a horn that applies ultrasonic energy contacts an anvil.

### Solution to Problem

The present invention provides the ultrasonic welding apparatus of independent claim 1 and the ultrasonic welding method of independent claim 12. The dependent claims specify preferred but optional features.

A main aspect of the invention to achieve the above is an ultrasonic welding apparatus that performs ultrasonic welding to a sheet-like member associated with an absorbent article,
the sheet-like member being transported while being wound onto an outer circumferential face of a rotating member,
the rotating member rotating about its central axis,
the apparatus, including:
the rotating member;
an ultrasonic processing unit
   that rotates about the central axis together with the rotating member, and
   that includes, as ultrasonic processing members,
      a horn that vibrates ultrasonically and
      an anvil that sandwiches the sheet-like member with the horn; and
an ultrasonic-energy regulating unit,
at least either one ultrasonic processing member of the horn and the anvil being guided so as to be able to reciprocate in a CD direction, the CD direction intersecting a transport direction of the sheet-like member,
the at least either one ultrasonic processing member of the horn and the anvil reciprocating in the CD direction with respect to a part of the sheet-like member, the part being wound around the rotating member,
in the reciprocation, the at least either one ultrasonic processing member of the horn and the anvil moving to a crossed-over position that is located beyond an end part of the sheet-like member in the CD direction, the horn and the anvil facing each other at the crossed-over position,
the outer circumferential face of the rotating member including sections in which each end part of the sheet-like member is to be positioned,
in the case where, of the sections, a section that the at least either one ultrasonic processing member of the horn and the anvil passes when starting to cross the sheet-like member in the CD direction being a first section, a section that the at least either one ultrasonic processing member of the horn and the anvil passes when finishing the crossing being a second section,
   the ultrasonic-energy regulating unit causing an amount (J/m) of ultrasonic energy to be applied per unit length in the CD direction to start decreasing, when the ultrasonic processing member is passing the second section.

Further, another aspect of the invention is an ultrasonic welding method in which a sheet-like member associated with an absorbent article is subjected to ultrasonic welding,
the sheet-like member being transported while being wound onto an outer circumferential face of a rotating member,
the rotating member rotating about its central axis,
the method including:
rotating an ultrasonic processing unit about the central axis together with the rotating member,
   the ultrasonic processing unit including, as ultrasonic processing members,
   a horn and
   an anvil facing the horn;
causing the horn to vibrate ultrasonically;
causing at least either one ultrasonic processing member of the horn and the anvil to reciprocate in a CD direction with respect to a part of the sheet-like member, the part being wound around the rotating member, the reciprocation being performed while the sheet-like member is being sandwiched between the horn and the anvil, and the CD direction intersecting a transport direction of the sheet-like member,
in the reciprocation, the at least either one ultrasonic processing member of the horn and the anvil moving to a crossed-over position that is located beyond an end part of the sheet-like member in the CD direction, the horn and the anvil facing each other at the crossed-over position,
the outer circumferential face of the rotating member including sections in which each end part of the sheet-like member is to be positioned,
in the case where, of the sections, a section that the at least either one ultrasonic processing member of the horn and the anvil passes when starting to cross the sheet-like member in the CD direction being a first section, a section that the at least either one ultrasonic processing member passes when finishing the crossing being a second section,
   causing an amount (J/m) of ultrasonic energy to be applied per unit length in the CD direction to start decreasing, when the ultrasonic processing member is passing the second section.

Other features of the invention will become clear from descriptions of this specification and attached drawings.

### Advantageous Effects of Invention

With this invention, weld residues can be prevented from remaining in the end edge of the sheet-like member in the CD direction, and a malfunction such as wear and breakage that may occur due to a horn that applies ultrasonic energy contacting an anvil can be prevented.

### Brief Description of Drawings

Fig. 1A is a schematic perspective view of a conventional ultrasonic welding apparatus 120. Fig. 1B is a diagram showing a state in which a sheet-like member 1a is wound around an outer circumferential face 130s of a rotating drum 130.
Fig. 2A is a schematic perspective view of a substrate 1a of a diaper 1 which has not been two-folded yet. Fig. 2B is a schematic perspective view of the two-folded substrate 1a immediately before being transported to an ultrasonic welding apparatus 20 according to a first embodiment.
Fig. 3 is a schematic perspective view of the ultrasonic welding apparatus 20 of the first embodiment as viewed obliquely from front above.
Fig. 4 is a schematic side view along arrows IV-IV in Fig. 3.
Fig. 5 is a schematic front view along arrows V-V in Fig. 3.
Fig. 6 is a partially cutaway, schematic side view of the ultrasonic welding apparatus 20 in which the substrate 1a and a rotating drum 30 are removed from Fig. 4.
Fig. 7A is a schematic perspective view of a support unit 73 as viewed obliquely from front and outside in a rotation-radius direction Dr30. Fig. 7B is a schematic perspective view of the support unit 73 as viewed obliquely from back and outside in the rotation-radius direction Dr30.
Fig. 8 is a schematic front view of the ultrasonic welding apparatus 20 and shows the ranges in a rotation direction Dc30 to which a forward movement and a backward movement are respectively allocated, the forward movement being movement in a forward path, the backward movement being movement in a return path.
Fig. 9 is a schematic perspective view of an anvil roller 71.
Fig. 10A is an explanatory diagram of a problem of an ultrasonic welding apparatus 20, and the diagram shows the substrate 1a wound around an outer circumferential face 30s of the rotating drum 30.
Fig. 10B is an explanatory diagram of a problem of an ultrasonic welding apparatus 20, and the diagram shows a substrate 1a wound around the outer circumferential face 30s of the rotating drum 30.
Fig. 10C is an explanatory diagram of a preferable method to be applied in a case where a horn 61 has a large inertia, and the diagram shows the substrate 1a wound around the outer circumferential face 30s of the rotating drum 30.
Fig. 11 is a schematic diagram of an ultrasonic processing unit 60a according to a second modified example as viewed in a rotation direction Dc30 of a rotating drum 30.
Fig. 12 is a schematic front view of an ultrasonic welding apparatus 20, and is a diagram to explain a cam curve according to a third modified example.
Fig. 13 is a schematic perspective view of an ultrasonic welding apparatus 20b according to a second embodiment as viewed obliquely from front above.
Fig. 14 is a schematic side view along arrows XIV-XIV in Fig. 13.
Fig. 15 is a schematic front view along arrows XV-XV in Fig. 13.
Fig. 16 is a schematic side view of the apparatus 20b, in which a substrate 1a and a rotating drum 30 are removed from Fig. 14.
Fig. 17A is a schematic perspective view of an ultrasonic processing unit 60b according to the second embodiment as viewed obliquely from front and outside in the rotation-radius direction Dr30, and Fig. 17B is a schematic perspective view of the unit 60b as viewed obliquely from back and outside.
Fig. 18 is a schematic perspective view of an ultrasonic processing unit 60c showing another example of the positional relation of a horn 61b and an anvil roller 71 according to the second embodiment.

### Description of Embodiments

At least the following matters will become clear from the description of this specification and attached drawings.

An ultrasonic welding apparatus that performs ultrasonic welding to a sheet-like member associated with an absorbent article,
the sheet-like member being transported while being wound onto an outer circumferential face of a rotating member,
the rotating member rotating about its central axis,
the apparatus, including:
the rotating member;
an ultrasonic processing unit
   that rotates about the central axis together with the rotating member, and
   that includes, as ultrasonic processing members,
      a horn that vibrates ultrasonically and
      an anvil that sandwiches the sheet-like member with the horn; and
an ultrasonic-energy regulating unit,
at least either one ultrasonic processing member of the horn and the anvil being guided so as to be able to reciprocate in a CD direction, the CD direction intersecting a transport direction of the sheet-like member,
the at least either one ultrasonic processing member of the horn and the anvil reciprocating in the CD direction with respect to a part of the sheet-like member, the part being wound around the rotating member,
in the reciprocation, the at least either one ultrasonic processing member of the horn and the anvil moving to a crossed-over position that is located beyond an end part of the sheet-like member in the CD direction, the horn and the anvil facing each other at the crossed-over position,
the outer circumferential face of the rotating member including sections in which each end part of the sheet-like member is to be positioned,
in the case where, of the sections, a section that the at least either one ultrasonic processing member of the horn and the anvil passes when starting to cross the sheet-like member in the CD direction being a first section, a section that the at least either one ultrasonic processing member of the horn and the anvil passes when finishing the crossing being a second section,
   the ultrasonic-energy regulating unit causing an amount (J/m) of ultrasonic energy to be applied per unit length in the CD direction to start decreasing, when the ultrasonic processing member is passing the second section.

With this ultrasonic welding apparatus of a sheet-like member associated with an absorbent article, the amount (J/m) of ultrasonic energy is caused to start decreasing when the ultrasonic welding member is passing the second section. Thus, reduction of melt that may occur in the downstream side in the traveling direction of the ultrasonic welding member that moves in the CD direction in the ultrasonic welding process can be achieved. As a result, weld residues can be prevented from jutting out as burrs in the end edge of the sheet-like member in the CD direction and remaining thereon.

Further, by causing to start decreasing the ultrasonic energy in the second section, the horn that ultrasonically vibrates directly hitting the anvil can be lessened at the crossed-over position that is located beyond the second section. Thus, wear and breakage of the horn and the anvil can be prevented and contamination of the sheet-like member from abrasion powder can be prevented.

An ultrasonic welding apparatus of a sheet-like member associated with an absorbent article, wherein preferably
the ultrasonic processing member, after passing to an outer section positioned outside than the second section in the CD direction, turns back at the crossed-over position as a turn back position,
when the ultrasonic processing member is passing the outer section before turning back at the turn back position, the ultrasonic-energy regulating unit further decreases the amount (J/m) of the ultrasonic energy.

With this ultrasonic welding apparatus of a sheet-like member associated with an absorbent article, when the ultrasonic processing member is passing the outer section positioned outside the second section, the amount (J/m) of ultrasonic energy is further decreased. Thus, wear and breakage of the horn and the anvil can be prevented and contamination of the sheet-like member with abrasion powder can be effectively prevented.

An ultrasonic welding apparatus of a sheet-like member associated with an absorbent article, wherein preferably
the ultrasonic-energy regulating unit causes the amount (J/m) of the ultrasonic energy to be applied per unit length to change by causing an amplitude of the ultrasonic vibration of the horn to change.

With this ultrasonic welding apparatus of a sheet-like member associated with an absorbent article, because the change of the amount (J/m) of ultrasonic energy is caused by changing the amplitude of the ultrasonic vibration, changing the amount (J/m) of ultrasonic energy can be performed easily and with high responsivity. In this way, ultrasonic energy can be made to start decreasing quickly in the second section.

An ultrasonic welding apparatus of a sheet-like member associated with an absorbent article, wherein preferably
a pressing mechanism is included, the pressing mechanism pressing with a pressing force one ultrasonic processing member of the horn and the anvil to another ultrasonic processing member, so as to sandwich the sheet-like member with the horn and the anvil, and
the pressing mechanism starts to decrease the pressing force while the amplitude is decreasing.

With this ultrasonic welding apparatus of a sheet-like member associated with an absorbent article, the pressing force is made to start decreasing while the amplitude is decreasing. Thus, with decreasing of the ultrasonic energy due to decreasing of the pressing force added to decreasing of the ultrasonic energy due to decreasing of the amplitude, as a result a decreasing amount (J/m/sec) of the ultrasonic energy per unit time can be made greater.

An ultrasonic welding apparatus of a sheet-like member associated with an absorbent article, wherein preferably
a space changing mechanism that changes a space between the horn and the anvil is included, and
the space changing mechanism starts to expand the space while the amplitude is decreasing.

With this ultrasonic welding apparatus of a sheet-like member associated with an absorbent article, the space can be made to start expanding while the amplitude is decreasing. Thus, with decreasing of the ultrasonic energy due to expanding the space added to decreasing of the ultrasonic energy due to decreasing of the amplitude, as a result a decreasing amount (J/m/sec) of ultrasonic energy per unit time can be made greater.

An ultrasonic welding apparatus of a sheet-like member associated with an absorbent article, wherein preferably
in the case where a section of the sheet-like member that the ultrasonic processing member crosses in the outer circumferential face of the rotating member is a crossing section,
the crossing section includes a first section that the ultrasonic processing member first passes in a forward path of the reciprocation and a second section that the ultrasonic processing member passes after the first section in the forward path,
when the ultrasonic processing member passes the second section in the forward path of the reciprocation, the ultrasonic energy is applied to a second part corresponding to the second section of the sheet-like member, to weld the second part, and
when the ultrasonic processing member passes the first section in a return path of the reciprocation, the ultrasonic energy is applied to a first part corresponding to the first section of the sheet-like member, to weld the first part.

With this ultrasonic welding apparatus of a sheet-like member associated with an absorbent article, time to return the ultrasonic energy that has been decreased when the ultrasonic processing member has finished crossing the sheet-like member to the ultrasonic energy of an amount necessary for the original welding can be ensured in the first section in the forward path and can be ensured in the second section in the return path. Thus, in the case where a long time is needed to increase the amplitude due to the inertia of the horn, the welded part can be formed to the sheet-like member without any problems.

An ultrasonic welding apparatus of a sheet-like member associated with an absorbent article, wherein preferably
a pressing mechanism is included, the pressing mechanism pressing with a pressing force one ultrasonic processing member of the horn and the anvil to another ultrasonic processing member, so as to sandwich the sheet-like member with the horn and the anvil, and
the ultrasonic-energy regulating unit causes the amount (J/m) of the ultrasonic energy that is applied per unit length to change by causing the amount of the pressing force to change.

With this ultrasonic welding apparatus of a sheet-like member associated with an absorbent article, the amount (J/m) of ultrasonic energy is caused to change by causing the amount of the pressing force to change. Thus, the ultrasonic energy can be certainly decreased in the second section.

An ultrasonic welding apparatus of a sheet-like member associated with an absorbent article, wherein preferably
the ultrasonic-energy regulating unit causes an amount (J/m) of the ultrasonic energy that is applied per unit length to change by causing a size of a space between the horn and the anvil to change.

With this ultrasonic welding apparatus of a sheet-like member associated with an absorbent article, the amount (J/m) of ultrasonic energy is caused to change by causing the size of the space between the horn and the anvil to change. Thus, the ultrasonic energy can be certainly decreased in the second section.

An ultrasonic welding apparatus of a sheet-like member associated with an absorbent article, wherein preferably
the ultrasonic-energy regulating unit causes an amount (J/m) of the ultrasonic energy that is applied per unit length to change, by causing a travel speed value at which the ultrasonic processing member moves in the CD direction to change.

With this ultrasonic welding apparatus of a sheet-like member associated with an absorbent article, the amount of the ultrasonic energy (J/m) is caused to change by causing the travel speed value at which the ultrasonic processing member moves in the CD direction to change. Thus, the ultrasonic energy can be certainly decreased in the second section.

An ultrasonic welding apparatus of a sheet-like member associated with an absorbent article, wherein preferably
the ultrasonic processing member that is either one of the horn and the anvil includes a roller member,
the roller member is provided rotatably and is arranged outside with respect to the outer circumferential face of the rotating member, and
the roller member moves in the CD direction while rolling on a rail-like member,
the rail-like member being provided extending in the CD direction not to move relative to the outer circumferential face of the rotating member,
the rail-like member serving as another ultrasonic processing member.

With this ultrasonic welding apparatus of a sheet-like member associated with an absorbent article, the rail-like member that serves as the other ultrasonic processing member is provided not to move relative to the outer circumferential face of the rotating member. Thus, the other ultrasonic processing member can maintain a relative positional relation with the sheet-like member wound around the outer circumferential face in a constant state. As a result, the ultrasonic welding process can be stabilized.

An ultrasonic welding apparatus of a sheet-like member associated with an absorbent article, wherein preferably
both of the horn and the anvil reciprocate in the CD direction while the sheet-like member is being sandwiched between the horn and the anvil.

With this ultrasonic welding apparatus of a sheet-like member associated with an absorbent article, both of the horn and the anvil move relative to the sheet-like member in the CD direction. Thus, at least weld residues that may attach and accumulate on the horn due to the ultrasonic welding process can be subsequently wiped off by contacting with the sheet-like member while the horn and the anvil are reciprocating in the CD direction. As a result, the accumulation of weld residues on the horn can be effectively prevented.

An ultrasonic welding method in which a sheet-like member associated with an absorbent article is subjected to ultrasonic welding,
the sheet-like member being transported while being wound onto an outer circumferential face of a rotating member,
the rotating member rotating about its central axis,
the method including:
rotating an ultrasonic processing unit about the central axis together with the rotating member,
   the ultrasonic processing unit including, as ultrasonic processing members,
   a horn and
   an anvil facing the horn;
causing the horn to vibrate ultrasonically;
causing at least either one ultrasonic processing member of the horn and the anvil to reciprocate in a CD direction with respect to a part of the sheet-like member, the part being wound around the rotating member, the reciprocation being performed while the sheet-like member is being sandwiched between the horn and the anvil, and the CD direction intersecting a transport direction of the sheet-like member,
in the reciprocation, the at least either one ultrasonic processing member of the horn and the anvil moving to a crossed-over position that is located beyond an end part of the sheet-like member in the CD direction, the horn and the anvil facing each other at the crossed-over position,
the outer circumferential face of the rotating member including sections in which each end part of the sheet-like member is to be positioned,
in the case where, of the sections, a section that the at least either one ultrasonic processing member of the horn and the anvil passes when starting to cross the sheet-like member in the CD direction being a first section, a section that the at least either one ultrasonic processing member passes when finishing the crossing being a second section,
causing an amount (J/m) of ultrasonic energy to be applied per unit length in the CD direction to start decreasing, when the ultrasonic processing member is passing the second section.

With this ultrasonic welding method of a sheet-like member associated with an absorbent article, an amount (J/m) of ultrasonic energy is caused to start decreasing, when the ultrasonic processing member is passing the second section. Thus, reduction of melt that may occur in the downstream side in the traveling direction of the ultrasonic welding member that moves in the CD direction in the ultrasonic welding process can be achieved. As a result, weld residues can be prevented from jutting out as burrs in the end edge of the sheet-like member in the CD direction and remaining thereon.

Further, by starting to decrease the ultrasonic energy in the second section, the horn that ultrasonically vibrates directly hitting the anvil can be lessened at the crossed-over position that is located beyond the second section. Thus, wear and breakage of the horn and the anvil can be prevented and contamination of the sheet-like member with abrasion powder can be prevented.

### === First Embodiment ===

An ultrasonic welding apparatus 20 according to the invention is an apparatus which forms a welded part 14 in a continuous sheet-like member 1a at intervals in the transport direction (e.g. a predetermined pitch P1), the sheet-like member 1a being transported in a production line. In this embodiment, the sheet-like member 1a is exemplified by a substrate 1a of pull-on disposable diapers 1.

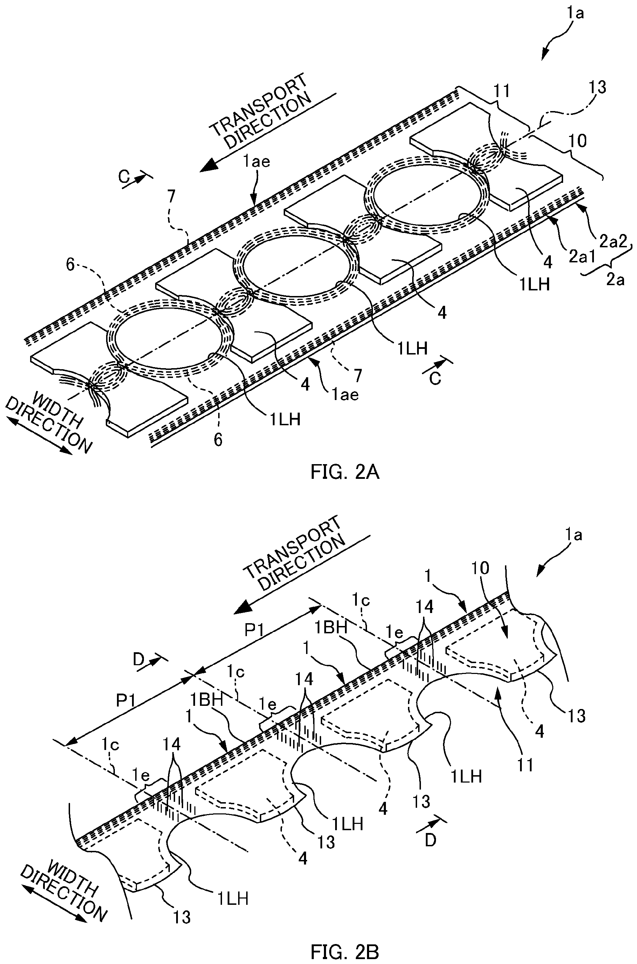

Fig. 2A and Fig. 2B are schematic perspective views and illustrate how the substrate 1a of the diaper 1 is transported to the ultrasonic welding apparatus 20. Fig. 2A shows the substrate 1a of the diaper 1 which has not been two-folded yet, and Fig. 2B shows the two-folded substrate 1a immediately before being transported to the ultrasonic welding apparatus 20.

The substrate 1a of the diaper 1 includes a continuous sheet 2a which continues in the transport direction. At this time shown in Fig. 2A, the substrate 1a is in a state in which, on a surface of the continuous sheet 2a which is to face wearer's skin, absorbent main bodies 4, 4... are placed on the substrate 1a with spacing of a product pitch P1 in the transport direction and joined to the substrate 1a with adhesive or the like.

In the substrate 1a at this time, leg openings 1LH are formed at a position between absorbent main bodies 4 and 4 which are adjacent to each other in the transport direction. Leg-circumference elastic members 6, which provide stretchability to the leg openings 1LH, are attached along the leg opening 1LH. In addition, waist-circumference elastic members 7, which provide stretchability to the waist opening 1BH, are attached along end edge parts 1ae and 1ae which are to be the waist opening 1BH.

The continuous sheet 2a is configured with a sheet 2a having a two layer structure, for example. That is, the continuous sheet 2a includes: a continuous sheet 2a1 (hereinafter referred to as an inner-layer sheet 2a1) which faces the skin side of a wearer to serve as an inner layer; and a continuous sheet 2a2 (hereinafter referred to as an outer-layer sheet 2a2) which faces the non-skin side of a wearer to serve as an outer layer. The inner-layer sheet 2a1 and the outer-layer sheet 2a2 are stacked in the thickness direction and are joined by such as adhesion or welding.

It is to be noted that an example of raw material of these inner-layer and outer-layer sheets 2a1 and 2a2 includes nonwoven fabric, woven fabric or a film made of a thermally weldable material such as thermoplastic resin. But, the invention is not limited thereto as long as the raw material is a material capable of being ultrasonically welded, that is, a material capable of being melted and joined by heat which is generated by friction due to application of ultrasonic energy.

The absorbent main body 4 is a component which absorbs excretion liquid, and the main body thereof is a body formed by shaping liquid absorbent fiber (e.g. pulp fiber) or liquid-absorbent particulate matter (e.g. super absorbent polymer) into a predetermined shape (e.g. a substantially hourglass shape). Such a shaped body is covered with a liquid-permeable cover sheet (not shown) such as tissue paper or nonwoven fabric. In addition, the shaped body is covered with a liquid-impermeable leak-proof sheet from the non-skin side.

Immediately before being fed into the ultrasonic welding apparatus 20, such a substrate 1a shown in Fig. 2A is two-folded in a crotch part 13, which is substantially the center of the substrate 1a in its width direction. Thus, the substrate 1a which has been two-folded as shown in Fig. 2B is transported to the ultrasonic welding apparatus 20. In other words, a part corresponding to a front piece 10 of a diaper 1 and a part corresponding to a back piece 11 are stacked in the up-down direction and are transported to the ultrasonic welding apparatus 20.

However, the substrate 1a of the diaper 1 at this time is in a state in which the part corresponding to the front piece 10 and the part corresponding to the back piece 11 are stacked but the above parts have not been joined yet. Accordingly, the ultrasonic welding apparatus 20 forms the welded part 14 of the substrate 1a by welding the substrate 1a on its part 1e which corresponds to waist-circumferential side-end parts 1e of a diaper 1. Consequently, the front piece 10 and the back piece 11 of the substrate 1a are joined.

Here, the parts 1e which are to be welded, that is, the parts 1e each of which corresponds to waist-circumferential side-end parts 1e of a diaper 1 are placed in the substrate 1a at a product pitch P1 in the transport direction on both sides of each absorbent main body 4. Accordingly, the ultrasonic welding apparatus 20 forms welded parts 14 on both side parts 1e of each absorbent main body 4 in the substrate 1a, at the product pitch P1 in the transport direction. At this time, as shown in Fig. 2B, for each of to-be-welded parts 1e, at least a pair of welded parts 14 and 14 is formed lined in the transport direction. Then, the substrate 1a in which these welded parts 14 are formed is transported to a downstream process, and in the downstream process, the substrate 1a is subsequently divided at a part 1c, which is between the pair of welded parts 14 and 14. Thereby, a diaper 1 having the waist opening 1BH and the leg openings 1LH is produced.

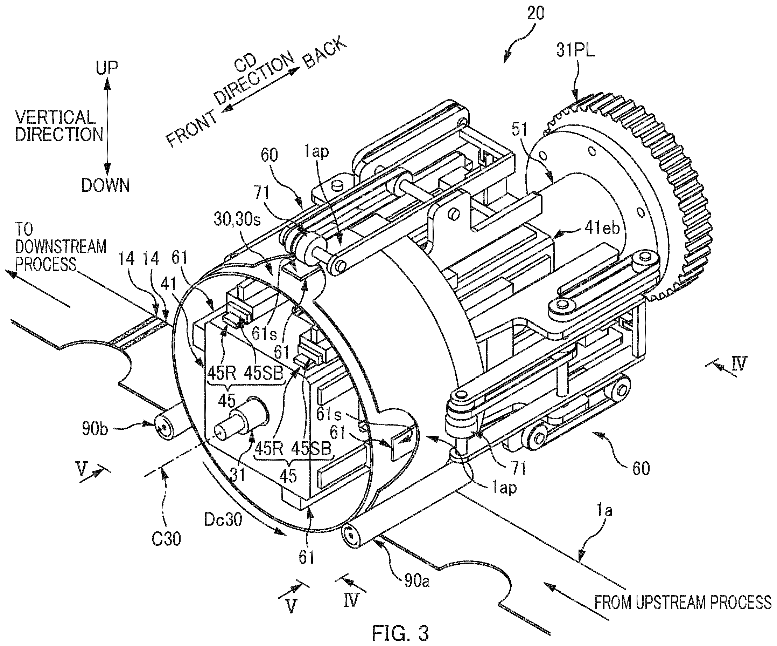

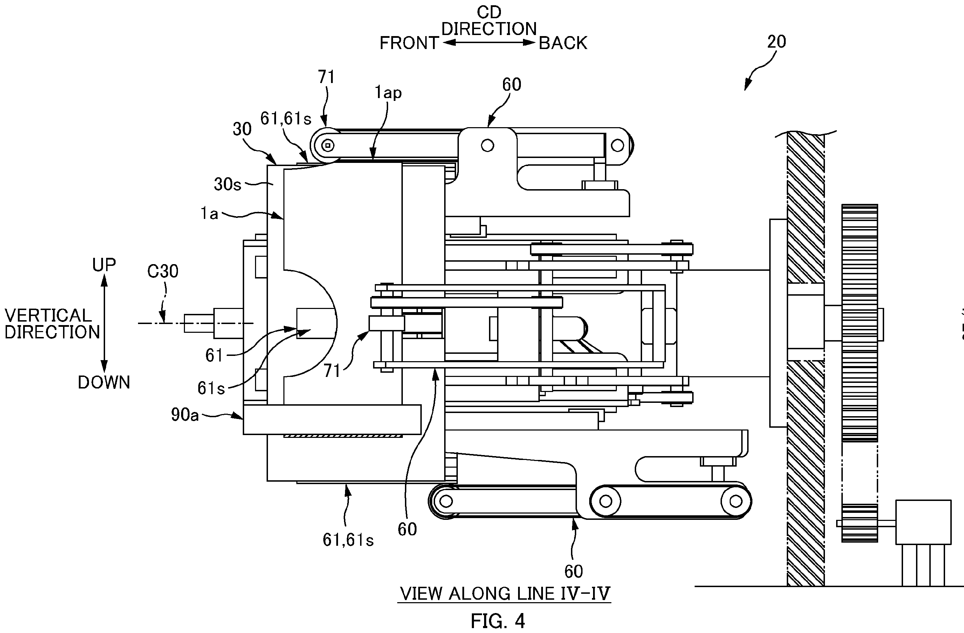

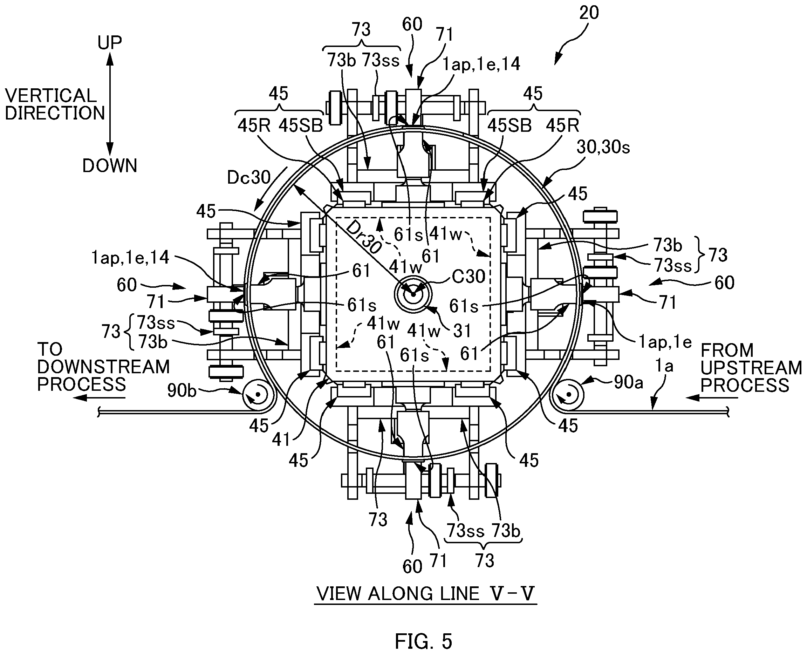

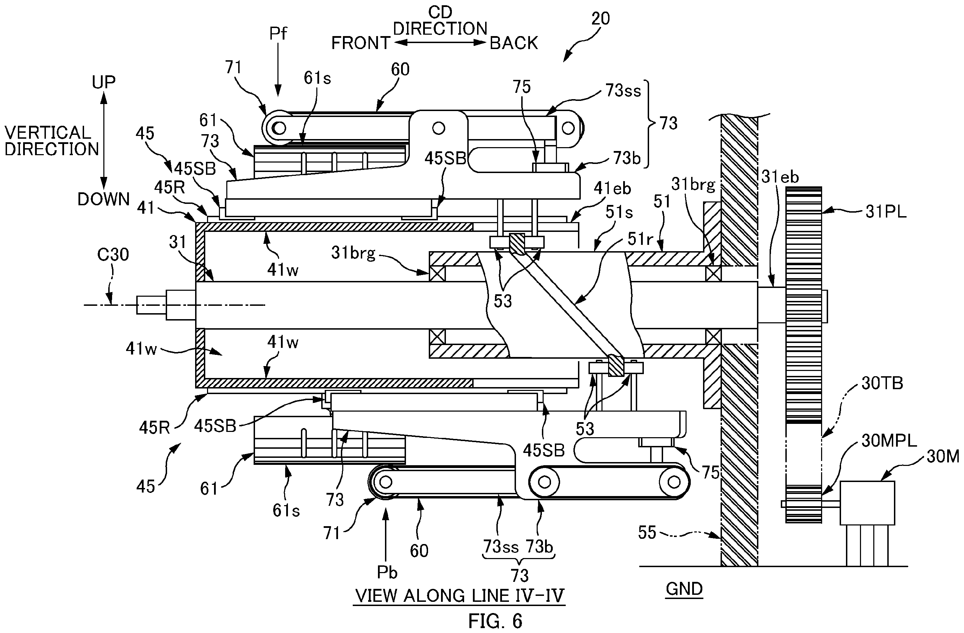

Fig. 3 is a schematic perspective view of the ultrasonic welding apparatus 20 as viewed obliquely from front above. Fig. 4 is a schematic side view along arrows IV-IV in Fig. 3, and Fig. 5 is a schematic front view along arrows V-V in Fig. 3. Fig. 6 is a schematic side view of the ultrasonic welding apparatus 20 with the substrate 1a and a rotating drum 30 are removed from Fig. 4, and some configuration of such as a column 41 illustrated in a cut-away view.

In the description below, a direction orthogonal to the transport direction of the substrate 1a in the production line is also referred to as a "CD direction". In this example, the CD direction is in the horizontal direction. A substrate 1a is being transported along a continuing direction in which the substrate 1a continues, and the width direction of the substrate 1a is designed to be parallel to the above-mentioned CD direction. The thickness direction of the substrate 1a is orthogonal to both of the continuing direction and the width direction of the substrate 1a.

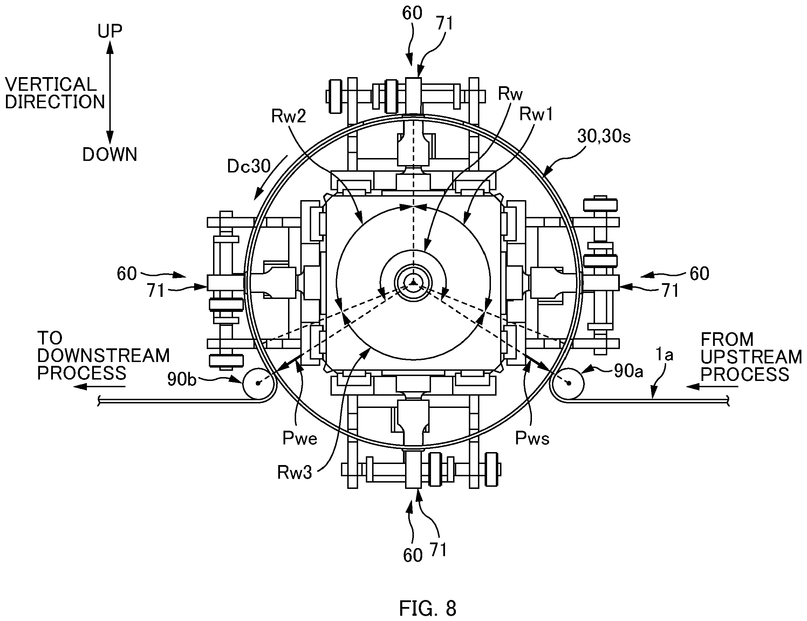

As shown in Fig. 3 and Fig. 5, the ultrasonic welding apparatus 20 includes the rotating drum 30 (corresponding to the rotating member), a plurality (four in this example) of ultrasonic processing units 60, 60 ..., and a pair of guide rolls 90a and 90b. The rotating drum 30 has a substantially cylindrical shape and rotates in one direction about a central axis C30 which is along the CD direction. The ultrasonic processing units 60, 60 ... rotate about the central axis C30 together with the rotating drum 30. Concerning the substrate 1a which is being transported from an upstream process, the pair of guide rolls 90a and 90b transports the substrate 1a to the downstream process while winding the substrate 1a on the outer circumferential face 30s of the rotating drum 30 through a predetermined range Rw in the rotation direction Dc30 (Fig. 8).

The rotating drum 30 is driven and rotated at substantially the same circumferential speed value (m/min) as the transport speed value (m/min) of the substrate 1a which is being transported from the upstream process . Accordingly, the substrate 1a is being transported along the outer circumferential face 30s of the rotating drum 30 while the substrate 1a is being wound on the outer circumferential face 30s, substantially without relative slippage to the substrate 1a. Then, after the substrate 1a has moved out of the predetermined range Rw, the substrate 1a is separated away from the outer circumferential face 30s and transported to the downstream process.

In the description below, for the purpose of explanation, it is assumed that the circumferential speed value (m/min) of the rotating drum 30 is constant. That is, in an actual production line, the circumferential speed value (m/min) of the rotating drum 30 may fluctuate. For example, when starting or stopping the production line, or when a sudden trouble occurs, the rotating drum 30 rotates at a circumferential speed value which is different from a constant circumferential speed value (m/min), which is a normal speed value. But, in most of the available time for producing the diapers 1, the rotating drum 30 rotates at a constant circumferential speed value (m/min), which is the above-mentioned normal speed value. On the other hand, the rotating drum 30 is rotating at an unsteady speed value, for a very short time like when starting the production line. Since the description based on the foregoing assumption will not disturb understanding the concept of the invention, the description is made below based on the foregoing assumption.

As shown in Fig. 3 and Fig. 5, each of the ultrasonic processing units 60, 60 ... is provided at every predetermined angle (e.g. 90°) in the rotation direction Dc30 of the rotating drum 30. Each ultrasonic processing unit 60 includes a horn 61 and a roller-like anvil 71 (corresponding to the roller member). The horn 61 vibrates ultrasonically and is arranged on the outer circumferential face 30s of the rotating drum 30 not to move relative to the outer circumferential face 30s. The anvil 71 is arranged outside with respect to the horn 61 in the rotation-radius direction Dr30 of the rotating drum 30 so that the substrate 1a is sandwiched between the anvil 71 and the horn 61.

Here, the horn 61 is in a rail-like shape extending in the CD direction, and the roller-like anvil 71 (hereinafter referred to as an anvil roller 71) is capable of rolling on such a rail-like horn 61 (corresponding to the rail-like member) . Thus, the anvil roller 71 is capable of reciprocating in the CD direction with respect to a part 1ap of the substrate 1a which is placed on the horn 61. Accordingly, during the reciprocation, ultrasonic energy is selectively applied from the horn 61 to the part 1ap of the substrate 1a which is sandwiched between the anvil roller 71 and the horn 61. Consequently, a welded part 14 is formed in the part 1ap of the substrate 1a.

The configuration of the ultrasonic welding apparatus 20 will be described in detail below.

As shown in Fig. 3, the main body of the rotating drum 30 is a cylinder, and its cross section in which its normal direction is the CD direction is in a circular shape, for example. At the circle center, which is the center of this cross section, a shaft member 31 is provided on the above-mentioned central axis C30 in a concentric and integrated manner. In addition, the shaft member 31 is supported rotatably by the bearing member 31brg shown in Fig. 6 while the axial direction of the shaft member 31 is oriented in the CD direction. Accordingly, the rotating drum 30 is capable of rotating about the above-mentioned central axis C30.

The rotating drum 30 is provided with rotational force by the servomotor 30M, which is a driving source, through a suitable rotational-force transmission mechanism. Accordingly, the rotating drum 30 is driven and rotated in one direction.

For example, in the example of Fig. 6, a so-called belt-type transmission device is used as the rotational-force transmission mechanism. That is, in the belt-type transmission device, an endless timing belt 30TB is wound around a pulley 31PL and a pulley 30MPL; the pulley 31PL is provided on the one end section 31eb of the shaft member 31 in a concentric and integrated manner, and the pulley 30MPL is provided on the driving rotational shaft of the servomotor 30M in a concentric and integrated manner. Thereby, driving rotational force generated by the servomotor 30M is transmitted to the shaft member 31, which serves as the central axis C30 of the rotating drum 30. Accordingly, the rotating drum 30 is driven and rotated by the servomotor 30M. A rotational-force transmission mechanism, however, is not limited thereto. For example, it is acceptable that the above-mentioned pulleys 31PL and 30MPL are respectively replaced with gears to configure the rotational-force transmission mechanism with a group of gears. In this example, though the cross section of the rotating drum 30 is in a circular shape, the invention is not limited thereto. For example, the shape of the cross section may be a polygon, such as a regular polygon having more corners than the number of the arranged ultrasonic processing units 60.

As shown in Fig. 5, a plurality (e.g. four) of the ultrasonic processing units 60, 60 ... are each provided at every predetermined angle in the rotation direction Dc30 of the rotating drum 30. The predetermined angle is set to an angle in which the length in the rotation direction Dc30 on the outer circumferential face 30s of the rotating drum 30 is substantially equal to a length corresponding to a single diaper. In the example of Fig. 5, the predetermined angle is set to 90°. Accordingly, the number of the ultrasonic processing units 60, 60 ... which are provided is four. The servomotor 30M as a driving source is controlled by a control section (not shown) such as a computer, a programmable logic controller (PLC), and the controlling is performed so that the rotating drum 30 rotates by the predetermined angle as the substrate 1a is fed from the upstream process by a length corresponding to a single diaper. Accordingly, each to-be-welded part 1e of the substrate 1a is correlated to one of the ultrasonic processing units 60 and the ultrasonic welding process is performed. Such a rotation is realized, for example, by controlling the position of the above-mentioned servomotor 30M based on synchronization signals.

The synchronization signals are outputted from a rotation detection sensor (not shown; e.g. a rotary encoder), which measures the transport amount of the substrate 1a in, for example, an apparatus which serves as a reference in the production line (e.g. a rotary die cutter apparatus which forms the leg opening 1LH of Fig. 2A by stamping). Such a synchronization signal is a rotational angle signal expressed by, for example, defining a transport amount corresponding to a single product diaper (substantially equal to the foregoing product pitch P1) as a unit transport amount and allocating the rotational angle values from 0° to 360° in proportion to the transportation amount. When the transportation is performed by an amount of a single diaper, each of the rotational angle values between 0° and 360° is outputted repeatedly and periodically. However, the synchronization signals are not limited to the rotational angle signals. For example, as the synchronization signals, digital signals may be used which are obtained by allotting each of digital values for 0 - 8191 to the above-mentioned unit transport amount in proportion to the transport amount.

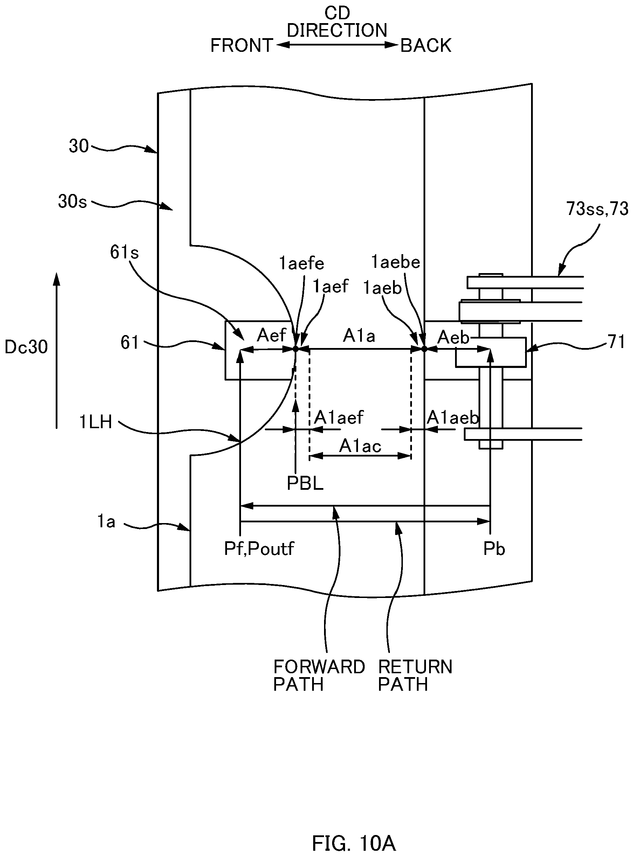

As illustrated in Fig. 3 and Fig. 5, each ultrasonic processing unit 60 includes the rail-like horn 61 and the anvil roller 71. The horn 61 extends along the CD direction and is fixed to the column 41 (to be described later) so as not to move relative to the outer circumferential face 30s of the rotating drum 30. The anvil roller 71 is provided so as to be able to reciprocate in the CD direction while rolling on the horn 61. As shown in Fig. 3, Fig. 5 and Fig. 6, the horn 61 includes a substantially flat surface 61s facing outwards in the rotation-radius direction Dr30 of the rotating drum 30, and the anvil roller 71 rolls on the substantially flat surface 61s. The substantially flat surface 61s serves as the oscillating surface 61s that vibrates ultrasonically. The oscillating surface 61s is fixed to the outer circumferential face 30s of the rotating drum 30 in a state in which the surface 61s coincides with the outer circumferential face 30s, or in which the surface 61s slightly protrudes outwards in the rotation-radius direction Dr30. The length of the oscillating surface 61s in the CD direction is designed to be a dimension in which the oscillating surface 61s extends beyond both sides of the substrate 1a in the CD direction, the substrate 1a being wound around the outer circumferential face 30s of the rotating drum 30 (e.g. see Fig. 10A) . Accordingly, based on reciprocation of the anvil roller 71 in the CD direction, the welded part 14 can be formed through the entire length of the substrate 1a in the CD direction.

The horn 61 is made of an appropriate metal such as an aluminum alloy, a titanium alloy, steel or the like. The horn 61 is connected to an oscillator via a booster and a converter (all of them are not shown). The oscillator has an electric circuit, and the electric circuit generates electric signals having a certain frequency from 20kHz to 35kHz when power is supplied from a suitable power supply. The converter converts the electric signals having the certain frequency which has been sent from the oscillator, into mechanical vibration having the same frequency, by means such as a piezo element. The booster amplifies the mechanical vibration sent from the converter and transmits it to the horn 61. Accordingly, the oscillating surface 61s of the horn 61 vibrates ultrasonically in the direction normal to the surface 61s.

Here, ultrasonic energy is applied to the substrate 1a using ultrasonic vibration of the oscillating surface 61s of the horn 61. If the frequency of the ultrasonic vibration is constant, the amount (J) of the ultrasonic energy can be changed by any of the following methods: changing the amplitude of the ultrasonic vibration of the oscillating surface 61s; or changing the magnitude (N) of force at which the substrate 1a is sandwiched between the oscillating surface 61s of the horn 61 and the anvil roller 71 (hereinafter referred to as the sandwiching force, or a pressing force) . For example, if the magnitude (N) of sandwiching force is constant, the resistance to the vibration increases/decreases in conjunction with increase/decrease of the amplitude. Consequently, the power consumption of the oscillator increases/decreases. Since most of the power consumption is applied to the substrate 1a as ultrasonic energy, ultrasonic energy that is applied to the substrate 1a increases/decreases in conjunction with increase/decrease of the amplitude. On the other hand, if the amplitude is constant, the resistance to the vibration increases/decreases in conjunction with increase/decrease of the magnitude (N) of sandwiching force. Consequently, the power consumption of the oscillator also increases/decreases, and most of the power consumption is applied to the substrate 1a as ultrasonic energy. That is, ultrasonic energy that is applied to the substrate 1a increases/decreases in conjunction with increase/decrease of the magnitude (N) of sandwiching force.

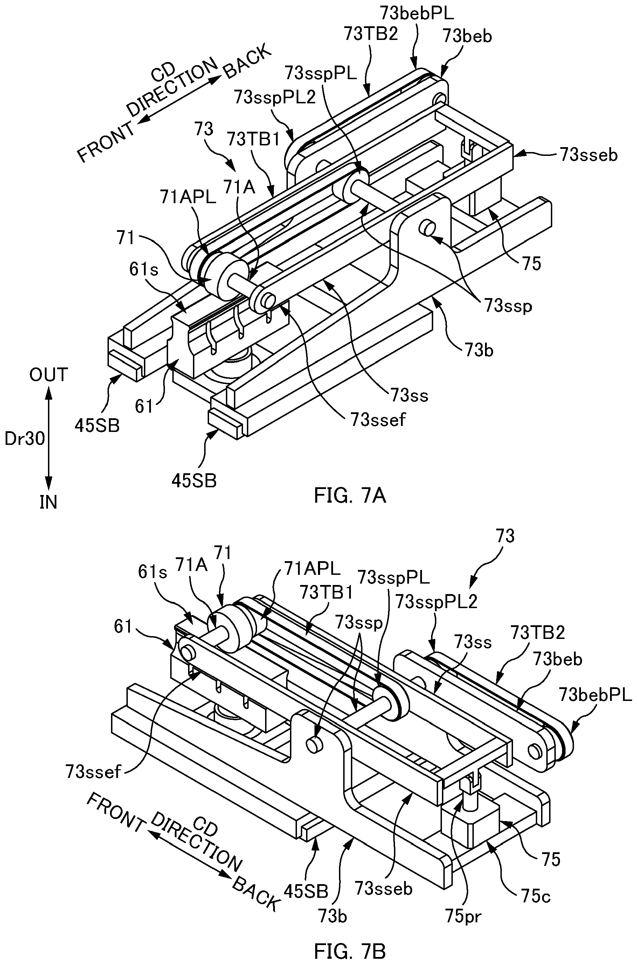

In the former case of changing the amplitude can be made by an oscillator. That is, the above-mentioned oscillator is capable of changing the amplitude of the ultrasonic vibration to any value, based on control signals transmitted from the ultrasonic-energy regulating unit (not shown), the ultrasonic-energy regulating unit being composed of a computer, a PLC, or the like. Further, in the latter case, changing the magnitude (N) of sandwiching force can be made by an air cylinder 75 (to be described later; see Fig. 7A and Fig. 7B) provided to the anvil roller 71. This will be described later. In this example, the oscillator is capable of regulating the amplitude of the ultrasonic vibration (the distance from the balanced position to the maximum displacement) to any value between 0 - 30 µm. However, the regulatable range of the amplitude is not limited thereto.

On the other hand, the anvil roller 71 is also made of an appropriate metal, such as steel, for example. As mentioned with reference to Fig. 5 and Fig. 6, the anvil roller 71 faces the oscillating surface 61s of the horn 61, and is arranged outside with respect to the oscillating surface 61s in the rotation-radius direction Dr30 of the rotating drum 30. The anvil roller 71 is provided so as to reciprocate in the CD direction while rolling on the oscillating surface 61s. For example, the reciprocation of the anvil roller 71 is realized as follows.

As shown in Fig. 3, Fig. 5 and Fig. 6, inside the rotating drum 30, a column 41 having a polygonal tubular shape is provided coaxially with the foregoing shaft member 31, which serves as the central axis C30 of the rotating drum 30. Most part of the column 41 is accommodated inside the rotating drum 30, and the one end section 41eb of the column 41 in the CD direction protrudes beyond the rotating drum 30. The column 41 is connected to and integrated with the shaft member 31 of the rotating drum 30 using a connecting member (not shown) . Accordingly, the column 41 rotates about the central axis C30 together with the rotating drum 30. In the description below, concerning the CD direction, a direction toward which the column 41 protrudes beyond the rotating drum 30 is called as "back", and the opposite direction is called as "front".

As shown in Fig. 5, a cross section of the column 41 (a cross section in which its normal direction is the CD direction) is a regular polygon having the same number of total corners as the number of arranged ultrasonic processing units 60, 60 ..., for example. Accordingly, the column 41 is a tubular body having the same number of wall sections 41w, 41w ... as the number of arranged ultrasonic processing units 60, 60 .... In the example of Fig. 5, the column 41 includes four ultrasonic processing units 60, 60 ..., and thus the column 41 has a square cross section. In other words, the column 41 is a square tubular body having four wall sections 41w, 41w .... The wall sections 41w respectively correlate to the ultrasonic processing units 60 one by one. That is, each wall section 41w includes a linear guide 45 for reciprocating the anvil roller 71 of the ultrasonic processing unit 60 in the CD direction. As shown in Fig. 6, the linear guide 45 includes a rail 45R which is fixed to the wall section 41w and extends in the CD direction, and sliding blocks 45SB and 45SB which are engaged with the rail 45R so as to be able to slide towards both sides in the CD direction. A support unit 73, which supports the anvil roller 71, is fixed to the sliding blocks 45SB and 45SB.

Fig. 7A and Fig. 7B are diagrams illustrating the support unit 73. Fig. 7A is a schematic perspective view of the support unit 73 as viewed obliquely from front above and outside in the rotation-radius direction Dr30, and Fig. 7B is a schematic perspective view of the support unit 73 as viewed obliquely from back and outside in the rotation-radius direction Dr30.

The support unit 73 includes a base section 73b fixed to the sliding blocks 45SB, 45SB ... and a seesaw-like member 73ss which is supported by the base section 73b so as to be capable of oscillating and extending in the CD direction. Here, the seesaw-like member 73ss is supported by the base section 73b with a support shaft 73ssp so as to be capable of oscillating, the support shaft 73ssp being provided in substantially the center in the CD direction. That is, the front-end part 73ssef and the back-end part 73sseb of the seesaw-like member 73ss are each capable of oscillating in the rotation-radius direction Dr30 of the rotating drum 30. More specifically, the front-end part 73ssef and the back-end part 73sseb move in substantially opposite directions to each other. In the front-end part 73ssef, the above-mentioned anvil roller 71 is supported rotatably. On the other hand, for example, a double-acting air cylinder 75 is provided in the back-end part 73sseb as a driving source for causing the seesaw-like member 73ss to oscillate.

As shown in Fig. 7B, the air cylinder 75 includes: a cylinder section 75c; a piston (not shown) which is capable of sliding inside the cylinder section 75c and which partitions the cylinder section 75c to form two pressure chambers; and a piston rod 75pr which is capable of coming out of and in the cylinder section 75c and which is integrated with the piston. The tip end of the piston rod 75pr is connected to the back-end part 73sseb of the seesaw-like member 73ss, and the cylinder section 75c is fixed to the base section 73b. Accordingly, by controlling the pressure (MPa) of compressed air being supplied to each of the two pressure chambers, it is possible, through movement of the piston rod 75pr, to press the anvil roller 71 against the oscillating surface 61s of the horn 61 and to separate the anvil roller 71 from the oscillating surface 61s.

For example, if one pressure chamber is opened to the atmosphere and compressed air is supplied to the other pressure chamber, the substrate 1a can be sandwiched between the anvil roller 71 and the oscillating surface 61s of the horn 61. In addition, if the supply pressure (MPa) of the compressed air changes, it is possible to regulate the magnitude (N) of the sandwiching force of the substrate 1a. It should be noted that a mechanism for regulating the supply pressure (MPa) of compressed air which is supplied to the air cylinder 75 is exemplified by a configuration including pressure-regulator valves (not shown) and directional control valves (e.g. solenoid valves; not shown), the pressure-regulator valves being respectively provided in channels through which compressed air is supplied to the pressure chambers, the directional control valves respectively switching connection and disconnection of the supply channels to a compressed-air source (not shown). However, the invention is not limited thereto.

On the other hand, rotation of the column 41 generates driving force to reciprocate the support unit 73 of the anvil roller 71 in the CD direction. That is, the column 41 rotates in the rotation direction Dc30 in an integrated manner with the rotating drum 30, and the ultrasonic welding apparatus 20 has a cam mechanism which drives the support units 73 by converting the rotation into reciprocation in the CD direction and transmitting it to the support units 73.

As shown in Fig. 6, such a cam mechanism has, for example, a cylindrical member 51 that is inserted into the column 41 in a coaxial manner with the column 41, and the cylindrical member 51 is fixed to a suitable support member 55 on the ground GND side so as not to rotate. A ribbed cam 51r is provided on the outer circumferential face 51s of the cylindrical member 51, and a pair of cam followers 53 and 53 is provided in the base section 73b of the support unit 73. The above-mentioned ribbed cam 51r is sandwiched between the pair of cam followers 53 and 53 in the front-back direction and is engaged with the cam followers 53 and 53. The ribbed cam 51r is provided in an endless manner continuously in the rotation direction Dc30 of the rotating drum 30. Further, the position of the cam 51r in the CD direction is changing according to the position of the cam 51r in the rotation direction Dc30, and a cam curve is therefore set. The setting of the cam curve determines the reciprocation of the support unit 73.

For example, in this example, under the condition that the circumferential speed value (m/min) of the rotating drum 30 is constant, the above-mentioned cam curve is set so that, in both of the forward path and the return path of reciprocation of the support unit 73, the support unit 73 moves at the same constant travel speed value (m/min) as each other.

More specifically, as shown in a schematic front view of Fig. 8, within a winding angular range Rw in which the substrate 1a is wound around the rotating drum 30, a first angular range Rw1 and a second angular range Rw2 are defined, and these angular ranges have the same magnitude and do not overlap each other. Here, forward movement, movement in the forward path, is allocated to a part of the cam curve which corresponds to the first angular range Rw1. For this purpose, that part has a shape in which the position of the ribbed cam 51r in the CD direction shifts forward in proportion to the change of the position of the ribbed cam 51r in the rotation direction Dc30. Accordingly, when the support unit 73 passes the first angular range Rw1, the support unit 73 and the accompanying anvil roller 71 move at a constant travel speed value (m/min) from a backward limit Pb to a forward limit Pf, the backward limit Pb being provided at the back in the CD direction (Fig. 6), the forward limit Pf being provided at the front (Fig. 6). Further, backward movement, movement in the return path, is allocated to a part of the cam curve which corresponds to the second angular range Rw2. For this purpose, that part has a shape in which the position of the ribbed cam 51r in the CD direction shifts backward in proportion to the change of the position of the ribbed cam 51r in the rotation direction Dc30. Accordingly, when the support unit 73 passes the second angular range Rw2, the support unit 73 and the accompanying anvil roller 71 move from the forward limit Pf to the backward limit Pb at the same travel speed value (m/min) as in the forward path.

In the example of Fig. 8, the first angular range Rw1 and the second angular range Rw2 are provided adjacent to each other. Accordingly, the anvil roller 71 that has reached the forward limit Pf as a result of the forward movement promptly turns and is to perform the backward movement. However, the invention is not limited thereto. For example, the anvil roller 71 may perform the backward movement after the anvil roller 71 stops for some period of time at the forward limit Pf.

In a state in which the anvil roller 71 is positioned at the backward limit Pb, the anvil roller 71 has completely finished crossing the substrate 1a, and is not in contact with the substrate 1a (see Fig. 10A, for example). The above-mentioned cam curve is set so that the anvil roller 71 stays at the backward limit Pb within an angular range Rw3 in the rotation direction Dc30, the angular range Rw3 being a range other than the first and second angular ranges Rw1 and Rw2. Accordingly, if a position of the initial end of the winding angular range Rw of the substrate 1a is defined as a winding start position Pws and a position of the final end of the winding angular range Rw is defined as a winding end position Pwe, the anvil roller 71 is positioned, at the backward limit Pb at both of the winding start position Pws and the winding end position Pwe. This allows the anvil roller 71 not to interrupt the following operations: winding operation of the substrate 1a to the outer circumferential face 30s of the rotating drum 30; and the releasing operation of the substrate 1a in the wound state from the outer circumferential face 30s.

Further, in the state where the anvil roller 71 is positioned in the forward limit Pf, the anvil roller 71 has completely finished crossing the substrate 1a and is not in contact with the substrate 1a (for example, refer to Fig. 10A). Accordingly, the forward limit Pf is positioned in a crossed-over position that is located beyond the front end part 1aef of the substrate 1a in the CD direction, and as is clear from above, the backward limit Pb is positioned in a crossed-over position that is located beyond the back end part 1aeb of the substrate 1a in the CD direction.

The anvil roller 71 rotates according to its reciprocation in the CD direction. That is, during the forward movement, the roller 71 is rotating forward at substantially the circumferential speed value (m/min) as the travel speed value (m/min) so as to roll forward. During the backward movement, the roller 71 is rotating backward at substantially the circumferential speed value (m/min) as the travel speed value (m/min) so as to roll backward. Accordingly, the anvil roller 71 is rolling on the substrate 1a and moving in the CD direction, substantially without relative slippage to the substrate 1a.

The foregoing rotation movements of the anvil roller 71 may be made without a driving source. For example, as shown in Fig. 7A and Fig. 7B, the anvil roller 71 is supported by the support units 73 through a suitable bearing member (not shown), and thus the anvil roller 71 is capable of rotating at an extremely small rotational resistance. The foregoing air cylinder 75 presses the anvil roller 71 against the oscillating surface 61s of the horn 61 via the substrate 1a. Accordingly, according to the reciprocation of the anvil roller 71, the anvil roller 71 obtains rotational force from the substrate 1a and rotates. Then, the anvil roller 71 rotates as a follower.

However, the foregoing rotation of the anvil roller 71 as a follower leads to a problem of reliability of the rotation. In the example of Fig. 7A and Fig. 7B, for a purpose of ensuring the rotation of the anvil roller 71, there is provided a movement converting mechanism which converts the reciprocation of the support unit 73 into rotational movement and transmits it the anvil roller 71. This movement converting mechanism is a so-called belt-type transmission mechanism.

That is, a pulley 71APL is fixed to a shaft section 71A which is integrated and concentric with the anvil roller 71, and another pulley 73sspPL is rotatably supported by the support shaft 73ssp of the seesaw-like member 73ss. An endless timing belt 73TB1 is wound around these pulleys 71APL and 73sspPL. To the latter pulley 73sspPL, another pulley 73sspPL2 is integrated and fixed in a concentric manner. In addition, another pulley 73bebPL is rotatably supported in a back-end part 73beb of the base section 73b of the support unit 73. Another endless timing belt 73TB2 is wound around these pulleys 73sspPL2 and 73bebPL. In addition, a part of the timing belt 73TB2 is connected to the column 41 through a connecting member, not shown.

Accordingly, when the support unit 73 is moving in the CD direction relative to the column 41, the anvil roller 71 is rotating by the amount of this movement. That is, when the support unit 73 moves forward, the anvil roller 71 rotates in the forward direction by the same amount as the amount of this forward movement. On the other hand, when the support unit 73 moves backward, the anvil roller 71 rotates in the backward direction by the same amount as the amount of this backward movement. This allows the anvil roller 71 to reliably roll on the substrate 1a during its reciprocation in the CD direction, substantially without relative slippage to the substrate 1a.

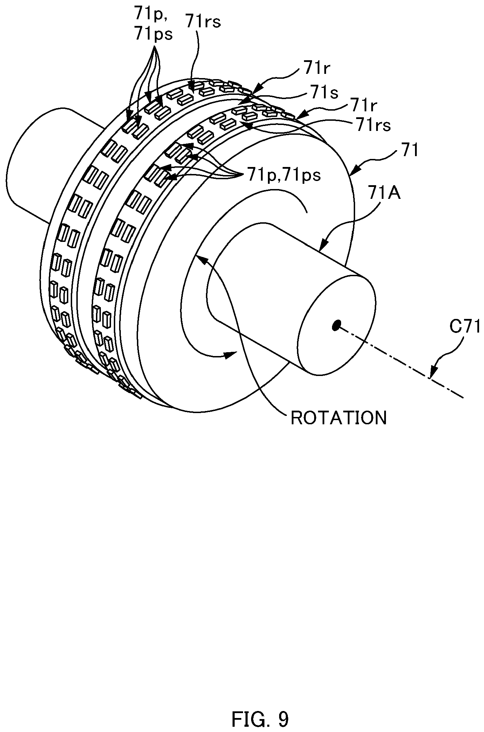

Fig. 9 is a schematic perspective view of the anvil roller 71. On the circumferential face 71s of the anvil roller 71, a plurality of protrusions 71p, 71p ... are provided for forming the welded parts 14 in a pattern on the substrate 1a. Specifically speaking, on the circumferential face 71s of the anvil roller 71, two endless ribs 71r and 71r are provided aligned in a direction along the rotational axis C71 of the anvil roller 71, and the ribs 71r and 71r extend through the entire length in its circumferential direction. On the top surface 71rs of each rib 71r, a plurality of island-shaped protrusions 71p, 71p ... are placed in a pattern in which there is a certain regularity. And, the top surfaces 71rs of the ribs 71r form low-compressed portions in the substrate 1a, and the top surfaces 71ps, 71ps ... of the plurality of protrusions 71p, 71p ... form high-compressed portions at a compression ratio larger than that for the low-compressed portions. Accordingly, the welded parts 14 are formed in a pattern in which there are a plurality of high-compressed portions.

In the case where ultrasonic welding process is performed to the substrate 1a while the anvil roller 71 is reciprocating in the CD direction, there are at least three cases of the timing to perform the welding process. Namely, the three cases are a first case in which ultrasonic welding process is performed in the forward path and is not performed in the return path, a second case in which ultrasonic welding process is not performed in the forward path and is performed in the return path, and a third case in which ultrasonic welding process is performed in both the forward path and the return path. Characteristic matters of the invention can be described, based on any of the cases. Thus, hereinbelow, the first case will be described, representing all the cases. According to the first case and the second case, ultrasonic welding process is performed in only either one of the forward path and the return path, thus the problem of unattractive appearance of welded parts 14 that may occur when the welded part 14 formed in the forward path and the welded part 14 formed in the return path are misaligned can be prevented in advance.

In the first case, when the anvil roller 71 is moving in the forward path, the horn 61 applies ultrasonic energy in an amount (J/m) necessary for welding, and in the return path the horn 61 applies ultrasonic energy in an amount (J/m) of a degree that does not cause welding. In this way, the welded part 14 of such as the above high-compressed portions is formed to the substrate 1a in mostly the forward path, and the welded part 14 is not formed in the return path. Here, the amount of the ultrasonic energy necessary for welding is defined with the size (J/m) of the ultrasonic energy applied per unit length in the CD direction. In this way, an appropriate amount of ultrasonic energy necessary for welding can be correctly applied.

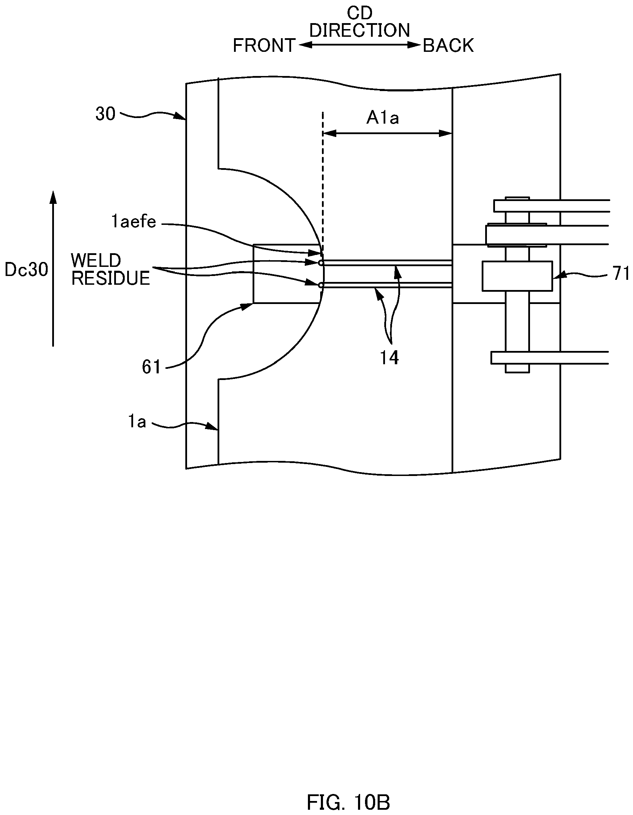

Fig. 10A and Fig. 10B are explanatory views of a problem of the ultrasonic welding apparatus 20. Both diagrams show the state where the substrate 1a is wound around the outer circumferential face 30s of the rotating drum 30.

As shown in Fig. 10A, in the case where a section of the substrate 1a along the CD direction to be crossed with the anvil roller 71 in the outer circumferential face 30s of the rotating drum 30 is defined as a "crossing section A1a", each path length of the forward path and the return path of the reciprocation of the anvil roller 71 is of course set to a length that juts out from both sides of the crossing section A1a in the CD direction. With this apparatus 20, the ultrasonic welding process is performed while the anvil roller 71 is moving in the forward path as described above. Thus, the melt that has melted from the substrate 1a tends to be pushed out to the front of the anvil roller 71 that moves forward in the CD direction.

Here, supposing that the ultrasonic energy of an amount necessary for welding is continuously applied to the crossed-over position Poutf that is located beyond the substrate 1a in the forward path, when the anvil roller 71 finishes crossing the front end edge 1aefe in the CD direction of the substrate 1a, the melt may jut out from the front end edge 1aefe and may harden. Thus, the melt residues jut out as burrs from the front end edge 1aefe and remain thereon, as shown in Fig. 10B, and accordingly there is a possibility that the appearance of the diaper 1 will become unattractive.

As can be understood from referring to Fig. 10A, the forward path and the return path in the reciprocation in the CD direction include sections Aef and Aeb other than the crossing section A1a. In other words, the forward path and the return path include outer sections Aef and Aeb positioned outer than the crossing section A1a in the CD direction. Hereinbelow, the outer section Aef positioned to the front in the CD direction is also referred to as a "front outer section Aef" and the outer section Aeb positioned to the back in the CD direction is also referred to as a "back outer section Aeb". Each of the outer sections Aef, Aeb does not include the substrate 1a, but because the horn 61 is a long rail shape in the CD direction, naturally the oscillating surface 61s of the horn 61 is included in the outer sections Aef and Aeb.

Thus, the horn 61 and the anvil roller 71 are in a contacting state in the outer sections Aef and Aeb. Here, in the case where the ultrasonic energy is continuously applied with the same amount as in the crossing section A1a, the horn 6 that ultrasonically vibrates to apply ultrasonic energy will directly hit the anvil roller 71. Then, the horn 61 and the anvil 71 may wear or break, or there is a possibility that the generated abrasion powder may attach to the substrate 1a and contaminate the substrate 1a. Malfunctions such as wear and breakage, may occur significantly particularly in the case where the horn 61 and the anvil roller 71 are both made of metal as in this example.

In order to solve the above problem, this ultrasonic welding apparatus 20 is devised as follows.

First, as shown in Fig. 10A, a section in which a back-end edge 1aeb that is one of the end part 1aeb of the substrate 1a in the CD direction in the crossing section A1a is defined as a "back inner section A1aeb" (corresponds to a first section), and a section in which a front end part 1aef that is the other end part 1aef of the substrate 1a in the CD direction in the crossing section A1a is defined as a "front inner section A1aef" (corresponds to a second section). When the sections are defined as above, the anvil roller 71 moving in the forward path moves from the backward limit Pb and passes the back outer section Aeb. Next, the anvil roller 71 passes the back inner section A1aeb adjacent to the back outer section Aeb, to cross the back end part 1aeb that is one end part 1aeb of the substrate 1a, and continues to cross a central part of the substrate 1a in the CD direction to reach the front inner section A1aef. The anvil roller 71 passes the front inner section A1aef, to cross the front end part 1aef that is the other end part 1aef of the substrate 1a, and finally passes the front outer section Aef adjacent to the front inner section A1aef to reach the forward limit Pf.

On the other hand, when the anvil roller 71 is passing the back outer section Aeb in this forward path, the ultrasonic energy of a first predetermined value (J/m) that is an amount in which welding cannot be performed is increased to a second predetermined value (J/m) that is an amount in which welding can be performed. The ultrasonic energy is maintained at the second predetermined value while the anvil roller 71 passes the back inner section A1aeb and crosses the central part of the substrate 1a to reach the front inner side section A1aef. In this way, the welded parts 14 are formed by the anvil roller 71 crossing most parts of the substrate 1a including the central part of the substrate 1a (for example, Fig. 10B).

In this example, however, when the anvil roller 71 is passing the front inner section A1aef, decreasing of the ultrasonic energy from the second predetermined value (J/m) to the first predetermined value (J/m) is started. Then, while the anvil roller 71 is passing the front end part 1aef of the substrate 1a, melting of the substrate 1a is prevented and generation of melts is prevented, resulting in prevention of melt residues that may jut out from the front end edge 1aefe of the substrate 1a and may remain as burrs. When the anvil roller 71 passes the front inner section A1aef in which the above phenomenon occurs, the anvil roller 71 passes the front outer section Aef, and while passing the front outer section Aef, the decreasing of the ultrasonic energy is still continuing, and until the forward limit Pf is finally reached, the size of the ultrasonic energy is decreased to the first predetermined value (J/m). Then, when the ultrasonic energy is substantially decreased to the first predetermined value (J/m), even in a situation where the substrate 1a is not between the anvil roller 71 and the horn 61, namely, even in a situation where the horn 61 is directly hitting the anvil roller 71, the horn 61 and the anvil roller 71 do not wear greatly or break. The anvil roller 71 that has reached the forward limit Pf starts the movement operation in the return path with the forward limit Pf as a turn back position, namely moves toward the back in the CD direction, but in the return path, the size of the ultrasonic energy is maintained at the first predetermined value until the anvil roller 71 reaches the backward limit Pb. Thus, ultrasonic welding process is not performed in the return path, and even when a situation in which the horn 61 directly hits the anvil roller 71 in the front outer section Aef and the back outer section Aeb in the return path, the horn 61 and the anvil roller 71 do not wear greatly or break.

An increasing operation of increasing an amount of the ultrasonic energy from the first predetermined value to the second predetermined value in the back outer section Aeb in the forward path, and a decreasing operation of decreasing an amount of the ultrasonic energy from the second predetermined value to the first predetermined value in the front inner section A1aef in the forward path is performed with the above described ultrasonic-energy regulating unit controlling the oscillator. The details are as described below.

To begin with, the ultrasonic-energy regulating unit is always monitoring the position of the anvil roller 71 in the CD direction during the reciprocation. This monitoring is performed indirectly, for example, based on signals outputted from a rotary encoder (not shown), which serves as a rotation detection sensor that detects the rotational angle of the rotating drum 30. That is, as mentioned above, the corresponding relation between the position of the rotating drum 30 in the rotation direction Dc30 and the position of the rotating drum 30 in the CD direction is uniquely predetermined based on the cam curve of the ribbed cam 51r. Accordingly, when the position of the anvil roller 71 in the rotation direction Dc30 of the rotating drum 30 is determined, it is possible to recognize the current position of the anvil roller 71 in the CD direction. On the other hand, the position of the anvil roller 71 in the rotation direction Dc30 can be detected based on the rotational angle value (0° to 360°) indicated by a signal outputted from the encoder.

Thus, data of values of rotational angles corresponding to predetermined positions included in the back outer section Aeb in the forward path are stored in the memory of the regulating unit. When the regulating unit detects that the rotational angle value indicated by a signal outputted from the encoder becomes equal to or larger than a rotational angle value in the memory, then, simultaneously or when a predetermined time has elapsed after the detection, the regulating unit starts to increase the amplitude from a small first amplitude corresponding to the first predetermined value to a second amplitude larger than the first amplitude, the second amplitude corresponding to the second predetermined value. In this way, the ultrasonic energy is increased from the above described first predetermined value (J/m) to the second predetermined value in the back outer section Aeb.

Similarly, data of the values of the rotational angles corresponding to predetermined positions included in the front inner section A1aef in the forward path is stored in the memory of the regulating unit. When the regulating unit detects that the rotational angle value indicated by a signal outputted from the encoder becomes equal to or larger than a rotational angle value in the memory, then, simultaneously or when a predetermined time has elapsed after the detection, the regulating unit starts to decrease the amplitude from the second amplitude corresponding to the second predetermined value to the first amplitude smaller than the second amplitude, the first amplitude corresponding to the first predetermined value. In this way, in the front inner section A1aef, the ultrasonic energy starts to decrease from the second predetermined value (J/m) to the first predetermined value (J/m), and the ultrasonic energy decreases to the first predetermined value (J/m) in the front outer section Aef.

The horn 61 that vibrates ultrasonically typically vibrates physically and also has inertia. Thus, when the amplitude is started to decrease, the vibrating continues with the inertia, thus there is a time lag when decreasing the ultrasonic energy, namely the decrease will be slightly delayed. Regarding this point, in this example, decrease of the ultrasonic energy is started in the front inner section A1aef in the forward path. Thus, even in the case where there is a time lag, the ultrasonic energy can be made to be a sufficiently decreased state, before the anvil roller 71 finishes crossing the front end edge 1aefe of the substrate 1a in the CD direction, and as a result, the weld residues remaining in the front end edge 1aefe and wear and breakage of the horn 61 and the anvil roller 71 due to contact of metals can be effectively prevented.

Of course, the second predetermined value (J/m) is set to a most appropriate value of ultrasonic energy needed to weld the substrate 1a, and the first predetermined value (J/m) is set to an amount in which wear and breakage of the anvil roller 71 and the horn 61 does not generally occur. The first and the second predetermined values are obtained by performing an actual apparatus experiment in which the ultrasonic processing apparatus 20 actually performs ultrasonic welding process of the substrate 1a. For example, the first predetermined value is determined with the level of the noise that occurs when the anvil roller 71 is hit with the horn 61 as a guideline. Further, the second predetermined value is determined by confirming whether or not there is a joint defect in the welded parts 14 formed to the substrate 1a or whether or not there is erosion.

The following guideline, however, can be shown regarding the first and the second predetermined values (J/m). In other words, the value of the second amplitude corresponding to the second predetermined value (J/m) is set to any value in a range from 15 µm to 30 µm. In this example, the amplitude is set to 30 µm. On the other hand, the value of the first amplitude corresponding to the first predetermined value (J/m) is set to any value in a range from 1 µm to 10 µm, and the value of the first amplitude is preferably set to any value in a range from 1 µm to 5 µm. In this example, the amplitude is set to 5 µm. The foregoing setting makes it possible to certainly form the welded parts 14 to the substrate 1a, and also certainly prevent the malfunctions such as wear and breaking of the horn 61 and the anvil roller 71.

Further, the predetermined position in which the amplitude is made to start decreasing in the front inner section A1aef is also determined by the actual apparatus experiment. In other words, potential positions of the above predetermined position is placed according to a plurality of levels in the CD direction, and after actually performing the ultrasonic welding process to the substrate 1a, the amount of the weld residues remaining in the front end edge 1aefe of the substrate 1a in the CD direction of the substrate 1a is confirmed, to determine the above predetermined position. As the above predetermined position, a position inside from a boundary position PBL (Fig. 10A) of the front inner section A1aef and the front outer section Aef by 2 mm to 10 mm in the CD direction can be exemplified. Preferably, the position is inside from the boundary position PBL by 3 mm to 6 mm. In this example, the position is inside from the boundary position PBL by 5 mm. In this way, remains of the weld residues on the front end edge 1aefe of the substrate 1a in the CD direction can be more effectively prevented.

In this example, the magnitude (N) of sandwiching force at which the substrate 1a is sandwiched between the oscillating surface 61s of the horn 61 and the anvil roller 71, namely the magnitude (N) of pressing force to press the anvil roller 71 to the oscillating surface 61s of the horn 61, is kept to be a constant value throughout the entire crossing section A1a in the forward path. Accordingly, by changing the amplitude, the amount (J/m) of ultrasonic energy can be reliably changed. The magnitude of the above pressing force (N) can also be obtained through the actual apparatus experiment as described above.

It is not limited to the above, however. Namely, in some cases, the magnitude (N) of the pressing force can be made to start decreasing in the front inner section A1aef. Further, this point of starting decreasing may be any point in time during the decreasing of the amplitude. In this way, decreasing of the ultrasonic energy due to decreasing of the amplitude, in addition to decreasing of the ultrasonic energy due to decreasing of the pressing force, can result in making the decreasing amount (J/m/secs) of ultrasonic energy per unit time large . In this way, the amount of the ultrasonic energy can be decreased to the first predetermined value (J/m) in a very short period of time. Needless to say, the pressing force that has been decreased in amount (N) is returned to the amount (N) before being decreased by the start of the next ultrasonic welding process. Further, the pressing mechanism that presses the anvil roller 71 to the horn 61 with the pressing force includes a seesaw-like member 73ss described above that supports the anvil roller 71, and an air cylinder 75 described above as a driving source that fluctuates the seesaw-like member 73ss. With the above described ultrasonic-energy regulating unit controlling the above described pressure regulating valve and the like, the supply pressure (MPa) of compressed air to the air cylinder 75 is regulated, and thus the decreasing operation of the amount of the above-described pressing force is performed.

Further, instead of the decreasing operation of the amount of the pressing force with the above-described air cylinder 75, or in addition to the decreasing operation, while the amplitude is decreasing, a space G between the anvil roller 71 and the horn 61 may be made to start expanding. In this way, the magnitude (N) of the pressing force to the horn 61, which is the sandwiching force, can be decreased.

The operation to expand the space G is performed by, for example, a space changing mechanism to change the space G between the horn 61 and the anvil roller 71. The space changing mechanism is, for example, a cam mechanism, and the space changing mechanism has a function to move the anvil roller 71 that has reached the front outer section Aef to outside in the rotational radius direction Dr30 of the rotating drum 30.

More specifically, the cam mechanism includes a cam follower (not shown) provided to the front end part 73ssef of the above-described seesaw-like member 73ss and a cam member (not shown) fixed to the outer circumferential face 30s of the rotating drum 30. The cam member includes, as a cam face, an inclined surface that is shaped with a projecting amount to the outside in the rotational radius direction Dr30 that has become larger the nearer to the outside in the CD direction. Further, the setting position of the cam member is a position in which the cam follower goes on a cam surface of the cam member when the anvil roller 71 reaches the front inner section A1aef. Accordingly, when the anvil roller 71 that has passed the central part of the substrate 1a passes the front inner section A1aef, the cam follower goes on the cam surface, thus the seesaw-like member 73ss is fluctuated so that the front end part 73ssef of the seesaw-like member 73ss moves to outside in the rotational radius direction Dr30 of the rotating drum 30, and the anvil roller 71 of the front end part 73ssef is also moved to outside in the rotational radius direction Dr30. As a result, the space G between the horn 61 and the anvil roller 71 is gradually expanded, and thus the magnitude (N) of the pressing force decreases. In this example, when the anvil roller 71 passes the front outer section Aef positioned to the outside than the front inner section A1aef in the CD direction, the anvil roller 71 and the horn 61 are in a spaced apart state from each other. When separated in this way, the wear and the breakage due to metal contact between the horn 61 and the anvil roller 71 that may occur in the front outer section Aef and the substrate 1a being contaminated due to generation of the metal abrasion powder and the like can be more effectively prevented. A similar cam member may also be provided to the back outer section Aeb.

In this example, the amplitude in the return path is maintained at the first amplitude that is smaller than the second amplitude, and thus the ultrasonic energy is to be applied in an amount (J/m) in which the substrate 1a does not generally get welded in the return path, but it is not limited thereto. For example, in also the return path, the amplitude may be changed with a changing pattern that is similar to that in the forward path, to change the amount (J/m) of the ultrasonic energy with a changing pattern that is similar to that in the forward path.

Namely, in the return path it may be as follows. First, when the anvil roller 71 is going backwards from the forward limit Pf and passing the front outer section Aef, the amplitude is started to increase from the first amplitude to the second amplitude. Then, when the anvil roller 71 is crossing the front inner section A1aef and the central part of the base 1a, the amplitude is maintained at the second amplitude. After that, when the anvil roller 71 is passing the back inner section A1aeb, the amplitude is made to start decreasing from the second amplitude to the first amplitude, then when the anvil roller 71 is passing the back outer section Aeb, such decreasing is continued. Finally, the amplitude is to be decreased to the first amplitude by the time the anvil roller 71 reaches the backward limit Pb.

Further, in some cases, in the forward path, the ultrasonic energy of the amount (J/m) necessary for welding is not applied and the ultrasonic energy may be applied in the return path. In that case, in the forward path, over the entire length of the forward path, the amplitude is maintained to an amplitude in which welding is not performed, that is the first amplitude, and in the return path the amplitude is to be changed with the above-described changing pattern.

As described above, because the horn 61 has inertia, to decrease the amplitude of the ultrasonic vibration from the second amplitude to the first amplitude, a certain amount of attenuation time is necessary. Further, similarly to this, in the case of increasing the amplitude from the first amplitude to the second amplitude, a certain amount of amplifying time is necessary. When the vibration is completely stopped as particularly in the case where the first amplitude is made to be 0 µm, a long period of time is necessary to restart the vibration and bring it back to the second amplitude, and in some cases there is a possibility that formation of the welded parts 14 will be hindered.

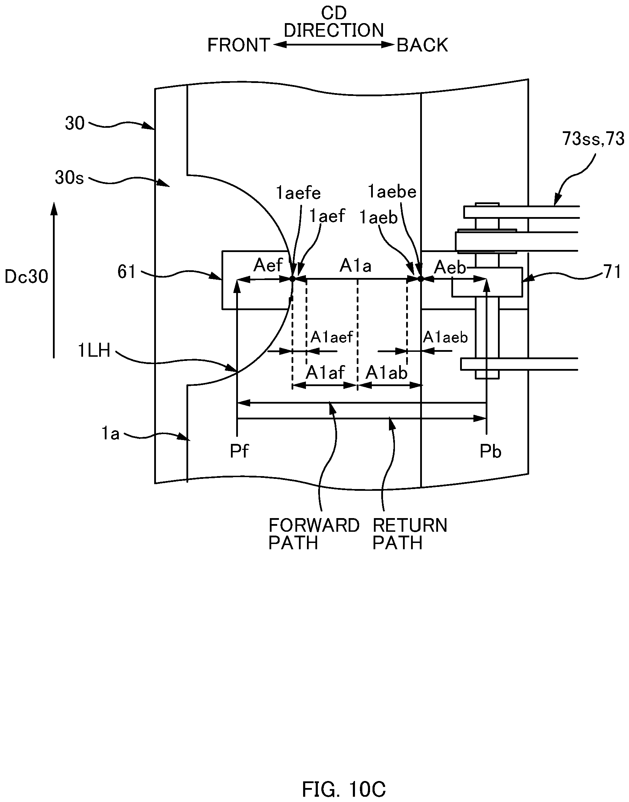

Fig. 10C is an explanatory view of a method of smoothly forming the welded parts 14 in the above case and shows the same format as in Fig. 10A. As shown in Fig. 10C, in this method, the welded parts 14 are formed partially in the forward path and in the return path. In other words, in the case where the crossing section A1a is divided into two to the front and the back in the CD direction and is partitioned to a front half section A1af (corresponds to a second section) and a back half section A1ab (corresponds to a first section), in the forward path the welded part 14 is formed when passing the front half section A1af and in the return path the welded part 14 is formed when passing the back half section A1ab. In this way, supposing that the amplitude is decreased to 0 µm in the front inner section A1aef and the front outer section Aef in the forward path, the time to bring back the amplitude to the second amplitude can be ensured in the front half section A1af in the return path, and in this way the welded parts 14 can be formed without any hindrance in the back half section A1ab in the return path. In this case, the amplitude is to be decreased to 0 µm in the back inner section A1aeb and the back outer section Aeb in the return path. Thus, it is necessary to bring back the amplitude to the second amplitude before reaching the front half section A1af in the next forward path, and the time necessary to return the second amplitude can be certainly ensured while the anvil roller 71 is passing the back half section A1ab in the next forward path.

### <<< First Modified Example >>>

In the first embodiment mentioned above, by changing the amplitude of the ultrasonic vibration, the amount (J/m) of ultrasonic energy is started to be increased from the first predetermined value (J/m) to the second predetermined value (J/m) in the back outer section Aeb in the forward path, and also the amount (J/m) of ultrasonic energy is started to be decreased from the second predetermined value (J/m) to the first predetermined value (J/m) in the front inner section A1aef in the forward path. In this regard, the first modified example is different from the first embodiment in that the amplitude is not changed over an entire length in the forward path and maintained at a constant value, and instead the magnitude (N) of the above-described sandwiching force, namely the magnitude (N) of the pressing force that presses the anvil roller 71 to the oscillating surface 61s of the horn 61, is changed to change the amount (J/m) of the ultrasonic energy with the above-described changing pattern.

The rest of the configuration in the first modified embodiment is substantially the same as that of above-described first embodiment. Thus, mainly the difference will be described below, and the same components as those of the first embodiment will be denoted by the same reference signs, and the description thereof will be omitted.

First, in the first modified example, the ultrasonic-energy regulating unit controls the oscillator, and thereby the amplitude of the ultrasonic vibration of the oscillating surface 61s of the horn 61 is maintained, for example, to a constant value of 30 µm. The regulating unit controls the foregoing pressure-regulator valve for the air cylinder 75, which is provided to the support unit 73. Accordingly the magnitude (N) of the pressing force changes as follows.

In other words, to begin with, when the anvil roller 71 is moving forward from the backward limit Pf and passing the back outer section Aeb in the forward path shown in Fig. 10A, the pressing force of a first load value (N) that is a magnitude in which welding cannot be performed is made to start increasing to the second load value (N) that is a magnitude in which welding can be performed. Then, the magnitude of the pressing force is maintained at the second load value (N) when the anvil roller 71 moves via the adjacent back inner section A1aeb and further crosses the central part of the substrate 1a to reach the front inner section A1aef. In this way, the welded parts 14 are formed when the anvil roller 71 crosses a large part of the substrate 1a including the central part of the substrate 1a.

The pressing force is made to start decreasing from the second load value (N) to the first load value (N), however, when the anvil roller 71 is passing the front inner section A1aef. Then, while the anvil roller 71 is passing the front end part 1aef of the substrate 1a, melting of the substrate 1a is restrained and generation of melt is restrained. As a result, weld residues that jut out from the front end edge 1aefe of the substrate 1a as burrs and remain thereon can be restrained. After the anvil roller 71 passes the front inner section A1aef in which such a phenomenon occurs, at the time of passing the front outer section Aef, the decrease of the pressing force is still continuing, and finally the magnitude of the ultrasonic energy is decreased to the first load value (N). When the magnitude is made to be small to the first load value, even in a situation in which there is the substrate 1a between the anvil roller 71 and horn 61, namely, in a situation in which the horn 61 directly hits the anvil roller 71, the horn 61 and the anvil roller 71 do not cause a large wear or breakage. The anvil roller 71 that has reached the forward limit Pf, starts the moving action of the return path with the forward limit PF as a turn back position, namely moves toward the back in the CD direction, and in the return path, the magnitude (N) of the pressing force is maintained to the first load value (N) until the anvil roller 71 reaches the backward limit Pb. Thus, ultrasonic welding process is not performed in the return path, and even if a situation in which the horn 61 directly hits the anvil roller 71 arises in the front outer section Aef and the back outer section Aeb in the return path, large wear or breakage of the horn 61 and the anvil roller 71 will not occur.

For example, the first load value (N) is set to any value within a range of 50% to 90% of the second load value (N). It is desirable that the first load value is set to any value within a range of 70% to 80%. This enables remaining of weld residues in the front end edge 1aefe and malfunctions such as wear and breakage of the horn 61 and the anvil roller 71 to be more certainly prevented.

In this example, the magnitude (N) of the pressing force in the return path is maintained to the first load value that is smaller than the second load value, and in this way in the return path the ultrasonic energy can be applied with an amount (J/m) in which the substrate 1a is substantially not welded, but it is not limited thereto.

For example, in the return path, by changing the magnitude (N) of the pressing force with a changing pattern that is similar to that in the forward path, the magnitude (J/m) of the ultrasonic energy may be changed with the similar changing pattern as in the forward path.

In other words, in the return path, it may be as follows. First, while the anvil roller 71 is moving back from the forward limit Pf and passing the front outer section Aef, the magnitude (N) of the pressing force is made to start increasing from the first load value to the second load value. Then, while the anvil roller 71 is crossing the front inner section A1aef and the central part of the substrate 1a, the magnitude (N) of the pressing force is maintained at the second load value. Thereafter, when the anvil roller 71 is passing the back inner section A1aeb, the magnitude (N) of the pressing force is made to start decreasing from the second load value to the first load value. Then, when the anvil roller 71 is passing the back outer section Aeb, the decreasing is continued, and finally before the anvil roller 71 reaches the backward limit Pb, the magnitude is decreased to the first load value.

In some cases, in the forward path, the ultrasonic energy of an amount (J/m) that is necessary for welding is not applied, and may be applied in the return path. In such a case, in the forward path, the magnitude of the pressing force is maintained to a magnitude (N) in which welding is not performed as in the first load value, along an entire length thereof, and in the return path, the magnitude (N) of the pressing force is to be changed with the above-described changing pattern.

### <<< Second Modified Example >>>

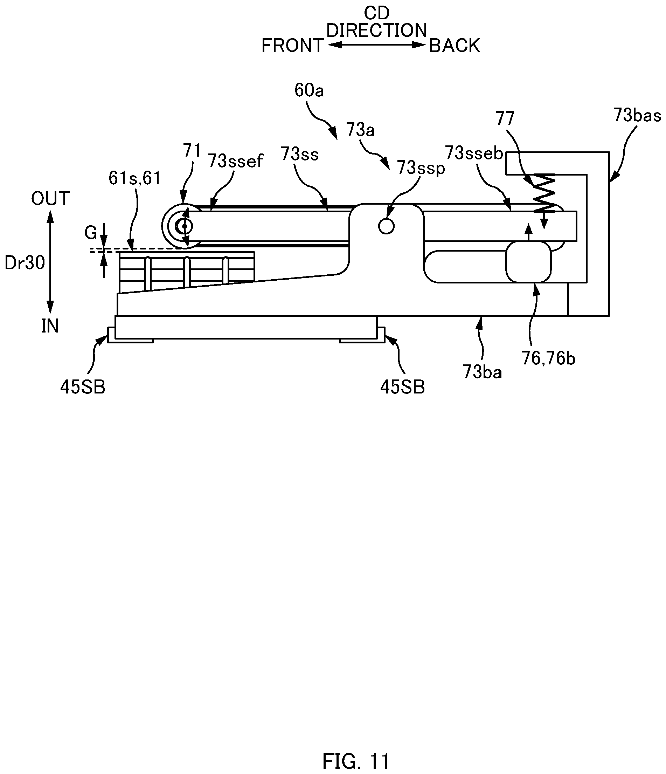

Fig. 11 is a schematic diagram of an ultrasonic processing unit 60a according to a second modified example as viewed from the rotation direction Dc30 of the rotating drum 30. In the second modified example, the size of the space G between the anvil roller 71 and the oscillating surface 61s of the horn 61 changes, and thereby, the amount (J/m) of ultrasonic energy is made to start increasing from the first predetermined value (J/m) to the second predetermined value (J/m) in the back outer section Aeb in the forward path, and in the front inner section A1aef in the forward path, the amount of ultrasonic energy is started to be decreased from the second predetermined value (J/m) to the first predetermined value (J/m).

That is, also in the second modified example, the amplitude is not changed over the entire length of the forward path and the return path and maintained as a constant value. But, the above-mentioned size of the space G changes with a predetermined changing pattern, as in the way the ultrasonic energy is changed with the above-described changing pattern. The details are as follows.

First, in the back outer section Aeb in the forward path, the size (µ) of the space G is started to be decreased from a size (µ) in which welding is not performed, which is a first space value, to a size (µ) in which welding is performed, which is a second space value. Then, when the anvil roller 71 moves via the adjacent back inner section A1aeb and further crosses the central part of the substrate 1a to reach the front inner section A1aef, the size of the space G is maintained to the second space value. In this way, the welded parts 14 are formed by crossing most parts of the substrate 1a including the central part of the substrate 1a.

On the other hand, when the anvil roller 71 is moving through the front inner section A1aef, the size of the space G is made to start increasing from the second space value to the first space value. In this way, the anvil roller gradually moves to a state in which welding is not performed, and when passing the front outer section Aef, the size of the space G further increases to be the first space value, and in this way, the anvil roller 71 and the horn 61 are in a separated state not touching each other. Then, the anvil roller 71 that has reached the forward limit Pf starts moving in the return path with the forward limit Pf as the turn back position, in other words the anvil roller 71 starts to move backwards in the CD direction, and in the return path the size of the space G is maintained to the first space value until the anvil roller 71 reaches the backward limit Pb. Thus, in the return path ultrasonic welding process is not performed in the return path, and a situation in which the horn 61 directly hits the anvil roller 71 does not occur in the front outer section Aef and the back outer section Aeb in the return path. Thus, large wear or breakage of the horn 61 and the anvil roller 71 does not occur.

Changing the size of the space G is realized, for example, by the following configuration. As shown in Fig. 11, in the second modified example, there are support units 73a each having substantially the same configuration as the support units 73 of the first embodiment. That is, the anvil roller 71 is supported rotatably by the front-end part 73ssef of the seesaw-like member 73ss. As mentioned above, the seesaw-like member 73ss is also rotatably supported by the support shaft 73ssp on the base section 73ba of the support unit 73a, the support shaft 73ssp being located at substantially the center in the CD direction. Accordingly, in the seesaw-like member 73ss, the front-end part 73ssef and the back-end part 73sseb can move in nearly opposite directions to each other. In other words, the seesaw-like member 73ss is capable of oscillating in a seesaw-like manner.

On the other hand, as driving sources for causing the seesaw-like member 73ss to oscillate, the support unit 73a of the second modified example includes: an air spring 76 that gives to the seesaw-like member 73ss a force in a direction in which the size of the space G is reduced; and a leaf spring 77 that serves as elastic member and that gives to the seesaw-like member 73ss a force in a direction in which the size of the space G expands.

More specifically, the air spring 76 has a bag body 76b that is substantially sealed. The bag body 76b inflates when internally pressurized by an air supply, and deflates when internally depressurized by ejection of air. The bag body 76b is placed between the base section 73ba of the support unit 73a and the back-end part 73sseb of the seesaw-like member 73ss. The bag body 76b is in contact with the back-end part 73sseb from inside in the rotation-radius direction Dr30 of the rotating drum 30. The leaf spring 77 is supported by a suitable stay member 73bas, which is disposed of the base section 73ba of the support unit 73a so as to be in contact with the back-end part 73sseb of the seesaw-like member 73ss from outside in the rotation-radius direction Dr30. Accordingly, the leaf spring 77 gives to the back-end part 73sseb of the seesaw-like member 73ss an elastic force being directed inwards in the rotation-radius direction Dr30.

Accordingly, the bag body 76b is internally pressurized by such means as supplying air to the inside of the bag body 76b and increasing its supply pressure (MPa) . Thereby, by inflation of the bag body 76b, the bag body 76b gives to the above-mentioned the back-end part 73sseb a force being directed outwards in the rotation-radius direction Dr30 of the rotating drum 30. When the magnitude of the force becomes larger than a force required to press and flatten the leaf spring 77 outwards in the rotation-radius direction Dr30, the seesaw-like member 73ss resists elastic force of the leaf spring 77 and simultaneously rotates so that the back-end part 73sseb moves outwards in the rotation-radius direction Dr30. The anvil roller 71 in the front-end part 73ssef moves inwards in the rotation-radius direction Dr30. Consequently, the size of the space G between the oscillating surface 61s of the horn 61 and the anvil roller 71 is reduced.

On the other hand, the bag body 76b is internally depressurized by such means as decreasing supply pressure (MPa) at which air is supplied inside the bag body 76b. Thereby, by deflation of the bag body 76b, a force that the bag body 76b gives to the back-end part 73sseb is reduced. When the force becomes smaller than a force required to press and flatten the leaf spring 77 outwards in the rotation-radius direction Dr30, the seesaw-like member 73ss rotates so that the back-end part 73sseb moves inwards in the rotation-radius direction Dr30. Thus, the anvil roller 71 in the front-end part 73ssef moves outwards in the rotation-radius direction Dr30, and the size of the space G between the horn 61 and the anvil roller 71 is consequently expanded.

A mechanism which supplies compressed air to the bag body 76b of the air spring 76 and which also ejects air from the same is exemplified by a configuration in which a compressed-air source (e.g. a compressor; not shown) is connected to the bag body 76b through a supply channel (e.g. pipework; not shown), and in which a pressure-regulator valve (not shown) is arranged in the supply channel. In this configuration, the ultrasonic-energy regulating unit controls the pressure-regulator valve, and thereby the size of the space G is changed. The mechanism for supplying and ejecting compressed air, however, is not limited thereto.

In the description above, the elastic member that gives a force in a direction in which the size of the space G is expanded is exemplified by the leaf spring 77. However, the invention is not limited thereto. For example, a disc spring or a coiled spring may also be employed. In addition, a bar-like member which undergoes elastic deflection deformation may be employed. For example, the one end section of the bar-like member is in contact with the back-end part 73sseb of the seesaw-like member 73ss from outside in the rotation-radius direction Dr30, and the bar-like member is supported on two parts except for the one end section by the base section 73ba of the support unit 73a so as not to rotate. When a force outwards in the rotation-radius direction Dr30 is applied to the back-end part 73sseb from the air spring 76, the bar-like member undergoes elastic deflection deformation, and thereby the size of the space G is changed.

A configuration in which the ultrasonic energy of the size (J/m) necessary for welding is to be applied in not only the forward path but also in the return path, and a configuration in which the ultrasonic energy is applied in the return path instead of the forward path can be achieved by one skilled in the art from the description in the first modified example. Thus, here description of such configurations will be omitted.

### <<< Third Modified Example >>>

In the third modified example, by changing the travel speed value (m/min) of the anvil roller 71 in the CD direction, the amount (J/m) of ultrasonic energy (J/m) is changed with the changing pattern as described above. In other words, other than when the anvil roller 71 is stopping at the backward limit Pb, both the magnitude (N) of the amplitude and the pressing force are not changed over substantially the entire length of the forward path and the return path, and are maintained at the second amplitude and the second load value as described above. The travel speed value (m/min) of the anvil roller 71, however, is changed with a predetermined changing pattern, in order to change the ultrasonic energy with the above-described changing pattern. The details will be as follows.

First, in the back outer section Aeb of the forward path, the travel speed (m/min) of the anvil roller 71 is made to start decreasing from a first travel speed value (m/min) that is large and in which welding is not performed to a second travel speed value (m/min) that is small and in which welding is performed. Then, the travel speed value is maintained at the second travel speed value (m/min) while the anvil roller 71 moves through the back inner section A1aeb and crosses the central part of the substrate 1a to reach the front inner section A1aef. In this way, the welded parts 14 are formed with the anvil roller 71 crossing most parts of the substrate 1a including the central part of the substrate 1a.

On the one hand, when the anvil roller 71 is passing the front inner section A1aef, the travel speed (m/min) is made to start increasing from the second travel speed (m/min) to the first travel speed (m/min) . Then, in this way, the state is gradually moved to a state in which welding is not performed, and when the anvil roller 71 is passing the front outer section Aef, the travel speed value (m/min) further increases to finally become the first travel speed value (m/min) . Here, when the anvil roller 71 is passing the front outer section Aef, the anvil roller 71 and the horn 61 will be in a contacting state with each other, and the anvil roller 71 will pass the front outer section Aef substantially at the large first travel speed value (m/min) as described above. Thus, the time period in which the horn 61 directly hits the anvil roller 71 can be made very short in the front outer section Aef, resulting in the horn 61 and the anvil roller 71 not causing a large wear or breakage.

Further, the anvil roller 71 that has reached the forward limit Pf immediately starts the movement in the return path with the forward limit Pf as the turn back position, namely, moves toward the back in the CD direction, but in the return path the travel speed (m/min) is substantially maintained to the first travel speed value (m/min) until the anvil roller 71 reaches the backward limit Pb. Thus, in the return path, the time period to cross the substrate 1a also is a very short period of time, thus ultrasonic welding process is not performed in the return path. Further, the passing time of the front outer section Aef and the back outer section Aeb in the return path also is a very short period of time, thus large wear and breakage of the horn 61 and the anvil roller 71 can be prevented.

When the anvil roller 71 has reached the backward limit Pb and is stopping at the backward limit Pb, the amplitude and the pressing force are decreased to a level in which malfunctions such as wear and breakage do not occur, for example, to the first amplitude and the first load value. Then, until the movement in the forward path is to be started again, the amplitude and the load value are increased to the above second amplitude and the second load value. In this way, the wear and breakage of the horn 61 and the anvil roller 71 which may occur in the backward limit Pb can be prevented.

The changing of the travel speed value (m/min) of the anvil roller 71 in the CD direction is made by the setting of the cam curve described above. That is, in this example, instead of the ultrasonic-energy regulating unit, which is used in the first embodiment and is configured of such as a computer, the above-described ribbed cam 51r and cam followers 53, 53 serve as the ultrasonic-energy regulating unit. The detailed description is as follows.

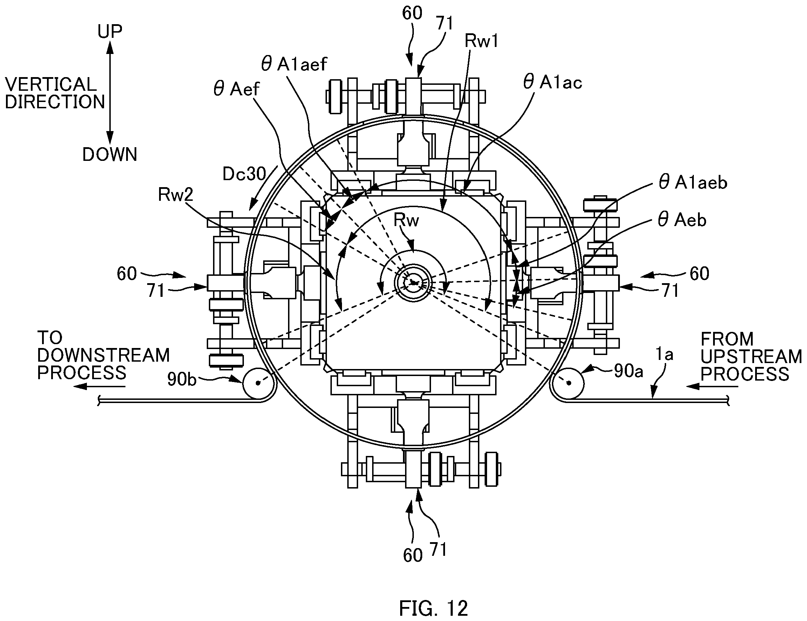

First, as mentioned above with reference to Fig. 8, in the cam curve of the cam 51r, the movement in the forward path (forward movement) is defined corresponding to the first angular range Rw1 in the rotation direction Dc30 of the rotating drum 30. Also, as shown in Fig. 10A, the forward movement includes movement in the back outer section Aeb, movement in the back inner section A1aeb, movement in a central section A1ac corresponding to parts of the substrate 1a other than both end parts 1aef, 1aeb, movement in the front inner section A1aef, and the movement in the front outer section Aef. Then, as shown in the schematically front view in Fig. 12, corresponding to each movement, angular ranges θAeb, θA1aeb, θA1ac, θA1aef, θAef, of a part of the first angular range Rw1 are each allotted without overlap. Then, the cam curve is formed in a pattern in which the anvil roller 71 shifts gear from the first travel speed value to the second travel speed value. Further, the cam curve is formed in a pattern in which the anvil roller 71 moves in the second travel speed value, in the angular range θA1aeb of the back inner section A1aeb and in the angular range θA1ac of the central section A1ac. Furthermore, the cam curve is formed in a pattern in which the anvil roller 71 shifts gear from the second travel speed value to the first travel speed value, in the angular range θA1aef of the front inner section A1aef and in the angular range θAef of the front outer section Aef.

On the other hand, the cam curve is also set with movement in the return path, which is a backward movement, corresponding to the second angular range Rw2 in the rotation direction Dc30 of the rotating drum 30. As is clear from referring to Fig. 12, this second angular range Rw2 is set smaller than the first angular range Rw1. The backward movement also includes the movement in the front outer section Aef, the movement in the front inner section A1aef, the movement in the central section A1ac, the movement in the back inner section A1aeb, and the movement in the back outer section Aeb. Then, corresponding to each movement, each of an angular range (not shown) of a part of the second angular range Rw2 is allotted without overlap. Then, the cam curve is formed in a pattern in which the anvil roller 71 moves in the first travel speed value in each of the angular range of the front outer section Aef, the angular range of the front inner section A1aef, the angular range of the central section A1ac, the angular range of back inner section A1aeb, and the angular range of back outer section Aeb.

In the first embodiment and the first to the third modified examples described above, the rail-like horn 61 is arranged so as not to move relative to the rotating drum 30, and the roller-like anvil 71, which serves as the anvil roller 71, is arranged outside in the rotation-radius direction Dr30 with respect to the horn 61, but the configuration is not limited to the above. For example, the following configuration may be employed. In other words, the rail-like anvil extending in the CD direction is fixed so as not to move relative to the rotating drum 30, and the roller-like horn (hereinafter, referred to as a horn roller) may be arranged outside with respect to the anvil in the rotation-radius direction Dr30. The configuration of the horn roller is the same as that disclosed in Japanese Unexamined Patent Application Publication (Translation of PCT Application) No. 10-513128, and the configuration is known. Accordingly, the detailed description will be omitted.

### === Second Embodiment ===

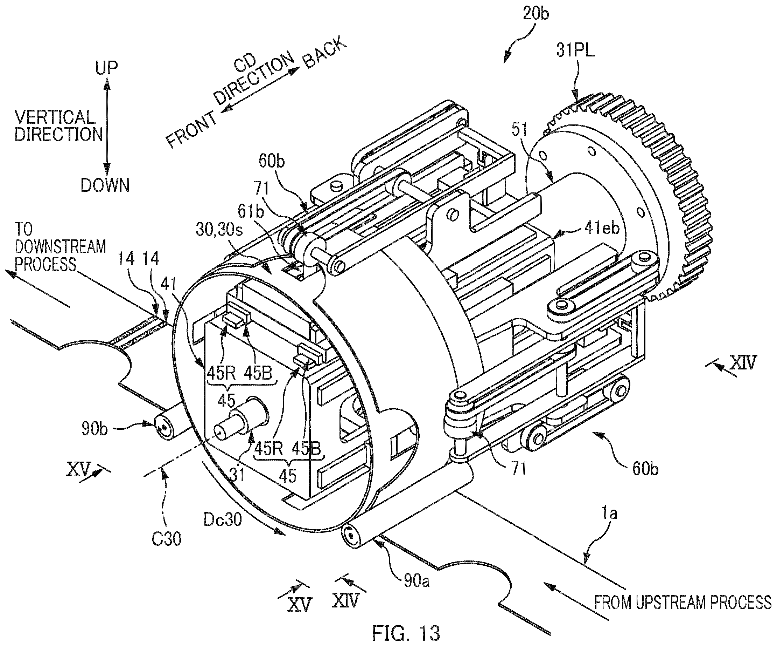

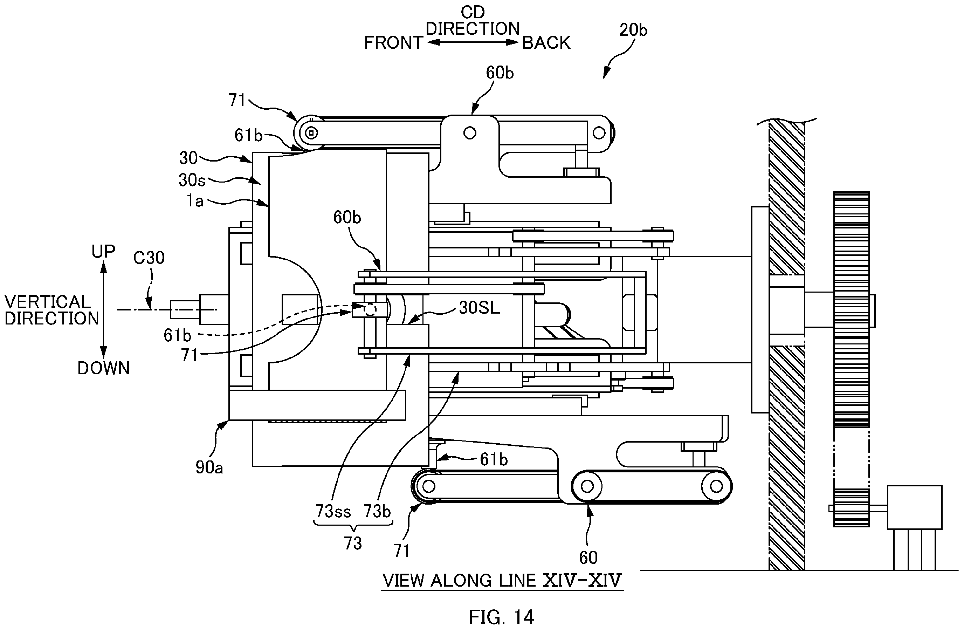

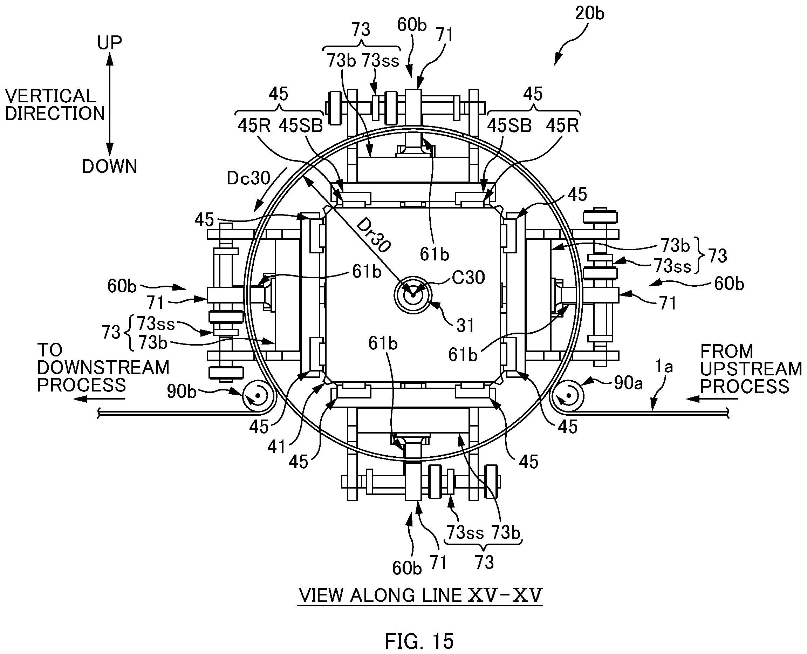

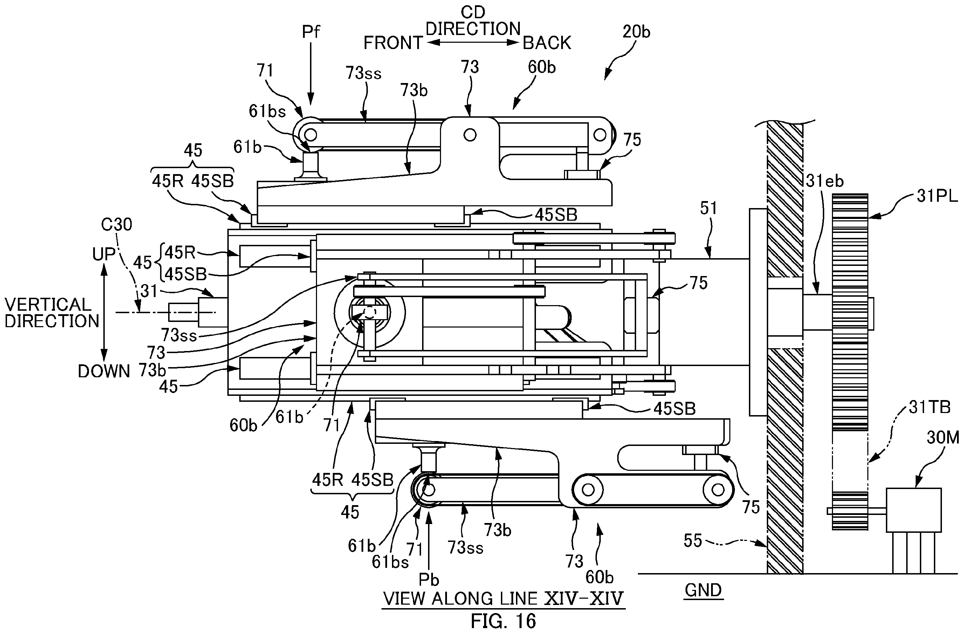

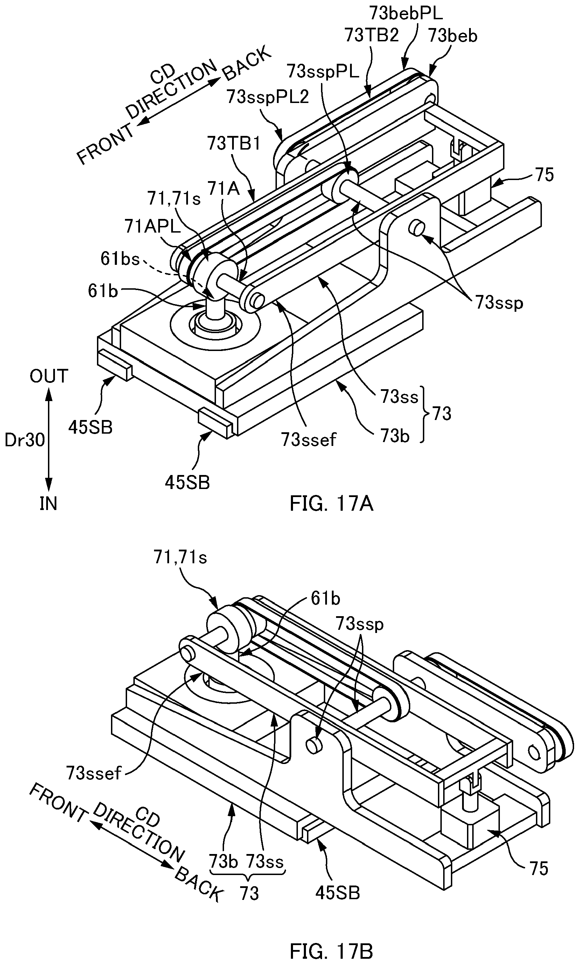

Fig. 13 to Fig. 17B are diagrams illustrating the ultrasonic welding apparatus 20b according to the second embodiment. Fig. 13 is a schematic perspective view of the ultrasonic welding apparatus 20b as viewed obliquely from front above. Fig. 14 is a schematic side view along arrows XIV-XIV in Fig. 13, and Fig. 15 is a schematic front view along arrows XV-XV in Fig. 13. Fig. 16 is a schematic side view of the apparatus 20b in which the substrate 1a and the rotating drum 30 are removed from Fig. 14. Fig. 17A is a schematic perspective view of the ultrasonic processing unit 60b as viewed obliquely from front and outside in the rotation-radius direction Dr30, and Fig. 17B is a schematic perspective view of the unit 60b as viewed obliquely from back and outside in the rotation-radius direction Dr30.

In the above described first embodiment, the horn 61 of the ultrasonic processing unit 60 is fixed to the column 41 so as not to move relative to the rotating drum 30, as shown in Fig. 5, thus, the horn 61 did not reciprocate in the CD direction as shown in Fig. 6. In this regard, this second embodiment differs mainly from the above-described first embodiment in that the horn 61b also performs the reciprocation in the CD direction, in cooperation with the reciprocation in the CD direction of the anvil roller 71, as shown in Fig. 16. The rest of the configuration is substantially the same as that of the first embodiment. Thus, mainly the difference will be described below. The same components as those of the first embodiment will be denoted by the same reference numerals, and the description thereof has been omitted.

As shown in Fig. 16, in this second embodiment, in addition to the anvil roller 71, the horn 61b is also supported with the support unit 73. Thus, the horn 61b is also capable of reciprocating in the CD direction. The anvil roller 71 and the horn 61b are configured to cooperate with each other so that the substrate 1a can be sandwiched therebetween. For example, in this example, the horn 61b is fixed to the base section 73b of the support unit 73. On the other hand, the anvil roller 71 is supported with the seesaw-like member 73ss. Accordingly, while ultrasonic energy is being applied to the substrate 1a from the oscillating surface 61bs of the ultrasonically vibrating horn 61b, and with the anvil roller 71 and the oscillating surface 61bs of the horn 61b cooperating with each other so that the substrate 1a is sandwiched therebetween, both of the anvil roller 71 and the horn 61b reciprocate in the CD direction. Consequently, a welded part 14 along the CD direction is formed in the substrate 1a.

In the above ultrasonic welding process to the substrate 1a, weld residue may adhere to and accumulate on the horn 61b that is not rotating in particular. But, in this example, not only the anvil roller 71, but also the horn 61b reciprocates. Accordingly, during its reciprocation, the horn 61b can wipe successively the weld residue attached thereto, by being in contact with the substrate 1a and by sliding on the substrate 1a. This makes it possible to effectively prevent accumulation of weld residue on the horn 61b.

Sandwiching the substrate 1a between the anvil roller 71 and the oscillating surface 61bs of the horn 61b is realized by moving of the anvil roller 71 in the rotation-radius direction Dr30 of the rotating drum 30 through oscillation of the seesaw-like member 73ss, the oscillation being caused by mainly the air cylinder 75. Accordingly, it is unnecessary to move the horn 61b in the rotation-radius direction Dr30 of the rotating drum 30. This makes it possible to stabilize ultrasonic vibration of the horn 61b, which is composed of precision devices.

Also in this second embodiment, as shown in Fig. 14, the main body of the rotating drum 30 is a cylinder part 30, which forms the outer circumferential face 30s. But, on the cylinder part 30, slit-shaped notches 30SL are formed extending from back to front in the CD direction, and the notches 30SL correspond to reciprocation paths in the CD direction of the anvil roller 71 and the horn 61b. Accordingly, there is a space at the position of each notch 30SL. That is, the inner circumferential side of the cylinder part 30 communicates with the outer circumferential side of the cylinder part. Accordingly, the anvil roller 71 and the horn 61b can reciprocate quickly in the CD direction without any interference with the cylinder part 30, while the substrate 1a is being sandwiched at the position of the notch 30SL between the anvil roller 71 and the horn 61b from both sides in the rotation-radius direction Dr30 of the rotating drum 30.

In the second embodiment, because the horn 61b also reciprocates in the CD direction together with the anvil roller 71, it is not necessary for the horn 61b to have a rail-like shape extending in the CD direction as described in the first embodiment, and the shape of the horn 61b may be selected freely. Accordingly, in the second embodiment, as shown in Fig. 17A, the shape of the horn 61b is a substantially cylindrical body 61b whose axial direction is oriented in the rotation-radius direction Dr30 of the rotating drum 30. A circular end surface 61bs of the substantially cylindrical body 61b serves as the oscillating surface 61bs that vibrates ultrasonically, and the end surface 61bs faces outwards in the rotation-radius direction Dr30 of the rotating drum 30. Consequently, the horn 61b is fixed to the base section 73b of the support unit 73 with the end surface 61bs facing the circumferential face 71s of the anvil roller 71.

The basic configuration of the ultrasonic welding apparatus 20b according to the second embodiment is substantially the same as that of the apparatus disclosed in Japanese Unexamined Patent Application Publication No. 2013-193450. Thus, the detailed description thereof has been omitted.

Based on the foregoing description, it is considered possible for persons skilled in the art to realize the invention by applying the configurations of the first to third modified examples of the first embodiment to the ultrasonic welding apparatus 20b of the second embodiment. Thus, the description thereof has been omitted.

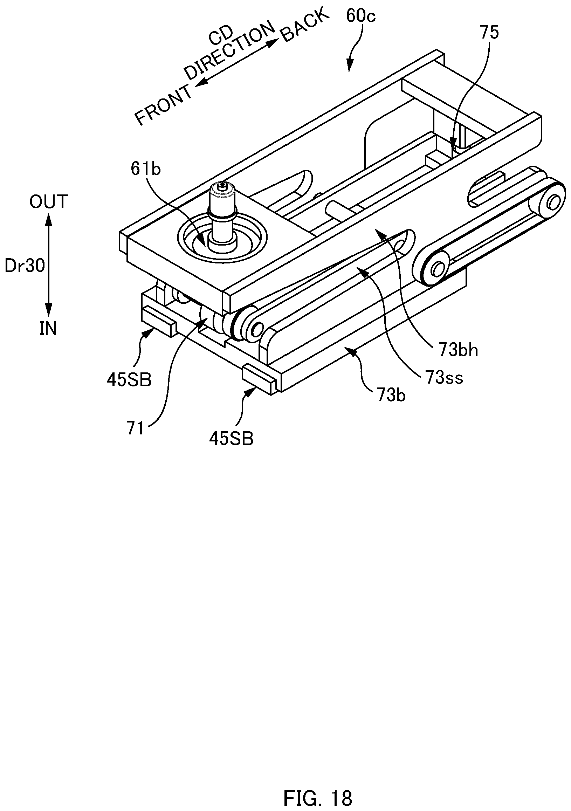

In addition, in the second embodiment, as shown in Fig. 17A, the horn 61b having a substantially cylindrical body is arranged inside with respect to the anvil roller 71 in the rotation-radius direction Dr30 of the rotating drum 30. The invention, however, is not limited thereto. For example, the positional relation in the rotation-radius direction Dr30 may be inverted. That is, the anvil roller 71 may be arranged inside with respect to the horn 61b in the rotation-radius direction Dr30, the horn 61b being a substantially cylindrical body. In this case, the front-end part 73ssef of the seesaw-like member 73ss supports the horn 61b, and the base section 73b supports the anvil roller 71. In some cases, however, an ultrasonic processing unit may be the ultrasonic processing unit 60c shown in the schematic perspective view of Fig. 18. That is, in this example, the anvil roller 71 is supported by the seesaw-like member 73ss. The foregoing base section 73b includes an extending part 73bh, which extends outwardly in the rotation-radius direction Dr30 beyond the position of the seesaw-like member 73ss. The extending part 73bh supports the horn 61b. This configuration may be employed.

### === Other Embodiments ===

While the embodiments of the invention are described above, the embodiments are for the purpose of elucidating the understanding of the invention and are not to be interpreted as limiting the invention. The invention can of course be altered and improved without departing from the gist thereof, and equivalents are intended to be embraced therein. The invention can be altered as described below, for example.

In the above-described first embodiment, for the purpose of facilitating explanation, the description is made based on an assumption that the circumferential speed value (m/min) of the rotating drum 30 is constant. But, as mentioned above, the circumferential speed value of the rotating drum 30 may change in actual use. In such a case, when the amplitude of the ultrasonic vibration increases/decreases, in cooperation with the increase/decrease of the circumferential speed value (m/min) of the rotating drum 30, the ultrasonic energy of an amount (J/m) necessary for welding can be applied irrespective of the change of the circumferential speed value of the rotating drum 30.

In the above-described first embodiment, as shown in Fig. 9, the anvil roller 71 includes two ribs 71r and 71r on the circumferential face 71s, and a plurality of protrusions 71p, 71p ... are provided in a pattern on the top surface 71rs of each rib 71r. The configuration, however, is not limited thereto. For example, the following configuration may be employed: two ribs along the CD direction are provided on the oscillating surface 61s of the rail-like horn 61 (shown in Fig. 6), and a plurality of protrusions are provided in a pattern on the top surface of each rib. In some cases, the above-mentioned ribs 71r and protrusions 71p, 71p ... may be provided on both of the anvil roller 71 and the horn 61. The rib 71r is not essential, that is, a plurality of protrusions 71p, 71p ... may be provided in a pattern directly on the circumferential face 71s of the anvil roller 71 or the oscillating surface 61s of the horn 61.

In the above-described foregoing embodiment, the disposable diaper 1 that is worn by a subject who wears the absorbent article and that absorbs excreted fluid is given as an example of the absorbent article. The invention, however, is not limited thereto. That is, as long as an absorbent article can absorb excreted fluid such as urine or menstrual blood, such can be the absorbent article according to the present invention. For example, sanitary napkins and pet sheets that absorb excreted fluid of pets are also included in the concept of absorbent articles according to the present invention.

In the above-described embodiment, the rotating drum 30 is exemplified as a rotating member. The configuration, however, is not limited thereto. That is, it is possible to use, without any problem, a member which is capable of rotating along the outer circumferential face 30s while holding the substrate 1a of the absorbent article 1 on the outer circumferential face 30s.

In the above-described embodiment, as shown in Fig. 3, the substrate 1a, which is an example of the sheet-like member 1a, is a continuous body in the transport direction. The configuration, however, is not limited thereto. For example, the substrate 1a may be a single-cut member having a size corresponding to a single diaper. In this case, however, it is preferable that a mechanism for actively holding the substrate 1a is provided on the outer circumferential face 30s of the rotating drum 30. For example, it is preferable that a plurality of suction holes are formed on the outer circumferential face 30s of the rotating drum 30 to attach the substrate 1a onto the outer circumferential face 30s by sucking from the suction holes.

In the above-described first embodiment, as shown in Fig. 6, a driving mechanism that reciprocates in the CD direction the support unit 73 associated with the anvil roller 71 is exemplified by the cam mechanism. The cam mechanism is exemplified by the followings: the ribbed cam 51r provided on the outer circumferential face 51s of the already discussed cylindrical member 51; and a pair of cam followers 53 and 53 which is provided in the base section 73b of the support unit 73 and which engage with the cam 51r by sandwiching it therebetween in the front-back direction. The configuration, however, is not limited thereto. For example, the following configuration is also acceptable: an endless grooved cam is provided based on the foregoing cam curve on the outer circumferential face 51s of the cylindrical member 51; a cam follower is provided on the base section 73b; and the cam follower fits into the grooved cam and engages with it.

In the foregoing embodiment, the CD direction, which intersects the transport direction of the substrate 1a, is exemplified by a direction orthogonal to the transport direction. The invention, however, is not limited thereto. That is, the CD direction may be slightly inclined to the direction orthogonal to the transport direction.

In the above-described embodiment, the section in which the anvil roller 71 crosses the substrate 1a along the CD direction in the outer circumferential face 30s of the rotating drum 30 that is the rotating member is defined as the "crossing section A1a" (refer to Fig. 10A). Here, when providing a supplementary explanation of this crossing section A1a, the crossing section A1a is "the section A1a corresponding to a position from which the anvil roller 71 starts crossing the substrate 1a in the CD direction to a position in which the anvil roller 71 ends crossing, in the state where the substrate 1a is wrapped around in a position designed with the outer circumferential face 30s of the rotating drum 30 without meandering". In other words, this crossing section A1a is "the section A1a sandwiched with the back end edge 1aebe and the front end edge 1aefe of the substrate 1a in the CD direction, in the state where the substrate 1a is wrapped around the above designed position".

### Reference Signs List

1 disposable diaper (absorbent article),
1BH waist opening,
1LH leg opening,
1a substrate (sheet-like member),
1ae end edge part,
1aef front end part (end part),
1aefe front end edge,
1aeb back end part (end part),
1aebe back end edge,
1ap part,
1c part,
1e side-end part (to-be-welded part),
2a continuous sheet,
2a1 inner-layer sheet,
2a2 outer-layer sheet,
4 absorbent main body,
6 leg-circumference elastic member,
7 waist-circumference elastic member,
10 front piece,
11 back piece,
13 crotch part,
14 welded part,
20 ultrasonic processing apparatus,
20b ultrasonic welding apparatus,
30 rotating drum (rotating member, cylinder part)
30s outer circumferential face,
30SL notch,
30M servomotor (driving source),
30MPL pulley,
30TB timing belt,
31 shaft member,
31brg bearing member,
31PL pulley,
31eb one end section,
41 column,
41eb one end section,
41w wall section,
45 linear guide,
45R rail,
45SB sliding block,
51 cylindrical member,
51s outer circumferential face,
51r ribbed cam,
53 cam follower,
55 support member on ground side,
60 ultrasonic processing unit,
60a ultrasonic processing unit,
60b ultrasonic processing unit,
60c ultrasonic processing unit,
61 horn (rail-like member, ultrasonic processing member),
61s oscillating surface,
61b horn (rail-like member, ultrasonic processing member),
61bs end surface (oscillating surface),
71 anvil roller (anvil, ultrasonic processing member),
71A shaft section, 71APL pulley,
71s circumferential face,
71r rib,
71rs top surface,
71p protrusion,
71ps top surface,
73 support unit,
73a support unit,
73TB1 timing belt,
73TB2 timing belt,
73b base section,
73ba base section,
73bas stay member,
73beb back-end part,
73bebPL pulley,
73bh extending part,
73ss seesaw-like member,
73ssef front-end part,
73sseb back-end part,
73ssp support shaft,
73sspPL another pulley,
73sspPL2 another pulley,
75 air cylinder,
75c cylinder section,
75pr piston rod,
76 air spring,
76b bag body,
90a guide roll,
90b guide roll,
A1a crossing section,
A1ac central section,
A1aeb back inner section (first section),
Aeb back outer section,
A1aef front inner section (second section),
Aef front outer section (outer section),
A1af front half section (second section),
A1ab back half section (first section),
Pb backward limit (crossed-over position located beyond end part, turn back position),
Pf forward limit (crossed-over position located beyond end part, turn back position),
Rw winding angular range,
Rw1 first angular range,
Rw2 second angular range,
Rw3 angular range except for first and second angular range,
Pws winding start position,
Pwe winding end position,
Poutf position,
PBL boundary position,
C30 central axis,
C71 rotational axis,
G space,
GND ground

## Claims

1. An ultrasonic welding apparatus (20) that performs ultrasonic welding to a sheet-like member (1a) associated with an absorbent article,
the sheet-like member being transported while being wound onto an outer circumferential face (30s) of a rotating member (30),
the rotating member rotating about its central axis (C30),
the apparatus, comprising:
the rotating member (30); and
an ultrasonic processing unit (60)
that rotates about the central axis (C30) together with the rotating member, and
that includes, as ultrasonic processing members,
a horn (61) that vibrates ultrasonically and
an anvil (71) that sandwiches the sheet-like member with the horn;
at least either one ultrasonic processing member of the horn (61) and the anvil (71) being guided so as to be able to reciprocate in a CD direction, the CD direction intersecting a transport direction of the sheet-like member,
the at least either one ultrasonic processing member of the horn and the anvil reciprocating in the CD direction with respect to a part (1ap) of the sheet-like member, the part being wound around the rotating member,
in the reciprocation, the at least either one ultrasonic processing member of the horn and the anvil moving to a crossed-over position (Pf, Pb) that is located beyond an end part (1aef, 1aeb) of the sheet-like member in the CD direction, the horn and the anvil facing each other at the crossed-over position,
the outer circumferential face (30s) of the rotating member including sections in which each end part of the sheet-like member is to be positioned,
**characterized in that** the apparatus further comprises an ultrasonic-energy regulating unit, and
in the case where, of the sections, a section that the at least either one ultrasonic processing member of the horn and the anvil passes when starting to cross the sheet-like member in the CD direction being a first section (A1aeb), a section that the at least either one ultrasonic processing member of the horn and the anvil passes when finishing the crossing being a second section (A1aef),
the ultrasonic-energy regulating unit causing an amount (J/m) of ultrasonic energy to be applied per unit length in the CD direction to start decreasing, when the ultrasonic processing member is passing the second section (A1aef).

2. An ultrasonic welding apparatus of a sheet-like member associated with an absorbent article according to claim 1, wherein
the ultrasonic processing member, after passing to an outer section (Aef) positioned outside than the second section (A1aef) in the CD direction, turns back at the crossed-over position as a turn back position,
when the ultrasonic processing member is passing the outer section before turning back at the turn back position, the ultrasonic-energy regulating unit further decreases the amount (J/m) of the ultrasonic energy.

3. An ultrasonic welding apparatus of a sheet-like member associated with an absorbent article according to claim 1 or 2, wherein
the ultrasonic-energy regulating unit causes the amount (J/m) of the ultrasonic energy to be applied per unit length to change by causing an amplitude of the ultrasonic vibration of the horn (61) to change.

4. An ultrasonic welding apparatus of a sheet-like member associated with an absorbent article according to claim 3, wherein
a pressing mechanism is included, the pressing mechanism pressing with a pressing force one ultrasonic processing member of the horn (61) and the anvil (71) to another ultrasonic processing member, so as to sandwich the sheet-like member with the horn and the anvil, and
the pressing mechanism starts to decrease the pressing force while the amplitude is decreasing.

5. An ultrasonic welding apparatus of a sheet-like member associated with an absorbent article according to claim 3 or 4, wherein
a space changing mechanism that changes a space (G) between the horn (61) and the anvil (71) is included, and
the space changing mechanism starts to expand the space while the amplitude is decreasing.

6. An ultrasonic welding apparatus of a sheet-like member associated with an absorbent article according to any one of claims 3 to 5, wherein
in the case where a section of the sheet-like member (1a) that the ultrasonic processing member crosses in the outer circumferential face (30s) of the rotating member is a crossing section (A1a),
the crossing section includes a first section (A1ab) that the ultrasonic processing member first passes in a forward path of the reciprocation and a second section (A1af) that the ultrasonic processing member passes after the first section in the forward path,
when the ultrasonic processing member passes the second section (A1af) in the forward path of the reciprocation, the ultrasonic energy is applied to a second part corresponding to the second section of the sheet-like member, to weld the second part, and
when the ultrasonic processing member passes the first section (A1ab) in a return path of the reciprocation, the ultrasonic energy is applied to a first part corresponding to the first section of the sheet-like member, to weld the first part.

7. An ultrasonic welding apparatus of a sheet-like member associated with an absorbent article according to claim 1 or 2, wherein
a pressing mechanism is included, the pressing mechanism pressing with a pressing force one ultrasonic processing member of the horn (61) and the anvil (71) to another ultrasonic processing member, so as to sandwich the sheet-like member with the horn and the anvil, and
the ultrasonic-energy regulating unit causes the amount (J/m) of the ultrasonic energy that is applied per unit length to change by causing the amount of the pressing force to change.

8. An ultrasonic welding apparatus of a sheet-like member associated with an absorbent article according to claim 1 or 2, wherein
the ultrasonic-energy regulating unit causes an amount (J/m) of the ultrasonic energy that is applied per unit length to change by causing a size of a space (G) between the horn (61) and the anvil (71) to change.

9. An ultrasonic welding apparatus of a sheet-like member associated with an absorbent article according to claim 1 or 2, wherein
the ultrasonic-energy regulating unit causes an amount (J/m) of the ultrasonic energy that is applied per unit length to change, by causing a travel speed value at which the ultrasonic processing member moves in the CD direction to change.

10. An ultrasonic welding apparatus of a sheet-like member associated with an absorbent article according to any one of claims 1 to 9, wherein
the ultrasonic processing member that is either one of the horn (61) and the anvil (71) includes a roller member,
the roller member is provided rotatably and is arranged outside with respect to the outer circumferential face (30s) of the rotating member, and
the roller member moves in the CD direction while rolling on a rail-like member,
the rail-like member being provided extending in the CD direction not to move relative to the outer circumferential face of the rotating member,
the rail-like member serving as another ultrasonic processing member.

11. An ultrasonic welding apparatus of a sheet-like member associated with an absorbent article according to any one of claims 1 to 9, wherein
both of the horn (61) and the anvil (71) reciprocate in the CD direction while the sheet-like member is being sandwiched between the horn and the anvil.

12. An ultrasonic welding method in which a sheet-like member (1a) associated with an absorbent article is subjected to ultrasonic welding,
the sheet-like member being transported while being wound onto an outer circumferential face (30s) of a rotating member (30),
the rotating member rotating about its central axis (C30),
the method comprising:
rotating an ultrasonic processing unit (60) about the central axis (C30) together with the rotating member (30),
the ultrasonic processing unit including, as ultrasonic processing members,
a horn (61) and
an anvil (71) facing the horn;
causing the horn to vibrate ultrasonically;
causing at least either one ultrasonic processing member of the horn and the anvil to reciprocate in a CD direction with respect to a part (1ap) of the sheet-like member, the part being wound around the rotating member, the reciprocation being performed while the sheet-like member is being sandwiched between the horn and the anvil, and the CD direction intersecting a transport direction of the sheet-like member,
in the reciprocation, the at least either one ultrasonic processing member of the horn and the anvil moving to a crossed-over position (Pf, Pb) that is located beyond an end part (1aef, 1aeb) of the sheet-like member in the CD direction, the horn and the anvil facing each other at the crossed-over position,
the outer circumferential face (30s) of the rotating member including sections in which each end part of the sheet-like member is to be positioned,
**characterized in that** in the case where, of the sections, a section that the at least either one ultrasonic processing member of the horn and the anvil passes when starting to cross the sheet-like member in the CD direction being a first section (A1aeb), a section that the at least either one ultrasonic processing member passes when finishing the crossing being a second section (A1aef),
causing an amount (J/m) of ultrasonic energy to be applied per unit length in the CD direction to start decreasing, when the ultrasonic processing member is passing the second section (A1aef).

## Patentansprüche

1. Ultraschall-Schweißvorrichtung (20), die Ultraschallschweißen an einem mit einem saugfähigen Artikel assoziierten flächigen Element (1a) ausführt,
wobei das flächige Element transportiert wird, während es auf eine Außenumfangsfläche (30s) eines rotierenden Elements (30) gewickelt ist,
wobei das rotierende Element um seine Mittelachse (C30) rotiert,
wobei die Vorrichtung Folgendes umfasst:
das rotierende Element (30); und
eine Ultraschall-Verarbeitungseinheit (60),
die zusammen mit dem rotierenden Element um die Mittelachse (C30) rotiert, und
die als Ultraschall-Verarbeitungselemente Folgendes umfasst:
ein Horn (61) das mit Ultraschallfrequenz schwingt, und
einen Amboss (71), der das flächige Element mit dem Horn klemmt;
wobei mindesten ein Ultraschall-Verarbeitungselement von dem Horn (61) und dem Amboss (71) derart geführt wird, dass es in der Lage ist, sich in einer CD-Richtung hin- und herzubewegen, wobei die CD-Richtung eine Transportrichtung des flächigen Elements kreuzt,
wobei sich das mindestens eine Ultraschall-Verarbeitungselement von dem Horn und dem Amboss in Bezug auf einen Teil (1ap) des flächigen Elements in der CD-Richtung hin- und herbewegt, wobei der Teil um das rotierende Element gewickelt ist,
wobei sich beim Hin- und Herbewegen das mindestens eine Ultraschall-Verarbeitungselement von dem Horn und dem Amboss zu einer Überquerungsposition (Pf, Pb) bewegt, die in der CD-Richtung hinter einem Endteil (1aef, 1aeb) des flächigen Elements liegt, wobei das Horn und der Amboss in der Überquerungsposition einander gegenüberliegen,
wobei die Außenumfangsfläche (30s) des rotierenden Elements Abschnitte umfasst, in denen jeder Endteil des flächigen Elements zu positionieren ist,
**dadurch gekennzeichnet, dass** die Vorrichtung weiter eine Ultraschallenergie-Reguliereinheit umfasst, und
im Fall, dass von den Abschnitten ein Abschnitt, den das mindestens eine Ultraschall-Verarbeitungselement von dem Horn und dem Amboss beim Beginn der Überquerung des flächigen Elements in der CD-Richtung passiert, um einen ersten Abschnitt (A1aeb) handelt, wobei ein Abschnitt, den das das mindestens eine Ultraschall-Verarbeitungselement von dem Horn und dem Amboss beim Abschließen der Überquerung passiert, um einen zweiten Abschnitt (A1aef) handelt,
wobei die Ultraschall-Reguliereinheit bewirkt, dass eine Menge (J/m) der pro Längeneinheit in der CD-Richtung aufzubringenden Ultraschallenergie beginnt, abzunehmen, wenn das Ultraschall-Verarbeitungselement den zweiten Abschnitt (A1aef) passiert.

2. Ultraschallenergie-Schweißvorrichtung eines mit einem saugfähigen Artikel assoziierten flächigen Elements nach Anspruch 1, wobei
das Ultraschall-Verarbeitungselement, nachdem es zu einem in der CD-Richtung gegenüber dem zweiten Abschnitt (A1aef) außen positionierten äußeren Abschnitt gelangt ist, an der Überquerungsposition als Umkehrposition umkehrt,
wenn das Ultraschall-Verarbeitungselement den äußeren Abschnitt passiert, bevor es an der Umkehrposition umkehrt, die Ultraschallenergie-Reguliereinheit die Menge (J/m) der Ultraschallenergie weiter verringert.

3. Ultraschallenergie-Schweißvorrichtung eines mit einem saugfähigen Artikel assoziierten flächigen Elements nach Anspruch 1 oder 2, wobei
die Ultraschallenergie-Reguliereinheit bewirkt, dass sich die Menge (J/m) der pro Längeneinheit aufzubringenden Ultraschallenergie ändert, indem sie bewirkt, dass sich eine Amplitude der Ultraschallschwingung des Horns (61) ändert.

4. Ultraschallenergie-Schweißvorrichtung eines mit einem saugfähigen Artikel assoziierten flächigen Elements nach Anspruch 3, wobei
ein Pressmechanismus enthalten ist, wobei der Pressmechanismus ein Ultraschall-Verarbeitungselement von dem Horn (61) und dem Amboss (71) mit einer Presskraft an ein anderes Ultraschall-Verarbeitungselement presst, um das flächige Element mit dem Horn und dem Amboss zu klemmen, und
der Pressmechanismus beginnt, die Presskraft zu verringern, während die Amplitude abnimmt.

5. Ultraschallenergie-Schweißvorrichtung eines mit einem saugfähigen Artikel assoziierten flächigen Elements nach Anspruch 3 oder 4, wobei
ein Zwischenraum-Änderungsmechanismus, der einen Zwischenraum (G) zwischen dem Horn (61) und dem Amboss (71) ändert, enthalten ist, und
der Zwischenraum-Änderungsmechanismus beginnt, den Zwischenraum auszudehnen, während die Amplitude abnimmt.

6. Ultraschall-Schweißvorrichtung eines mit einem saugfähigen Artikel assoziierten flächigen Elements nach einem der Ansprüche 3 bis 5, wobei
im Fall, dass es sich bei einem Abschnitt des flächigen Elements (1a), den das Ultraschall-Verarbeitungselement in der Außenumfangsfläche (30s) des rotierenden Elements quert, um einen Querungsabschnitt (A1a) handelt,
der Querungsabschnitt einen ersten Abschnitt (A1ab), den das Ultraschall-Verarbeitungselement auf einer Vorwärtsbahn der Hin- und Herbewegung zuerst passiert, und einen zweiten Abschnitt (A1af), den das Ultraschall-Verarbeitungselement nach dem ersten Abschnitt auf der Vorwärtsbahn passiert, umfasst,
wenn das Ultraschall-Verarbeitungselement den zweiten Abschnitt (A1af) auf der Vorwärtsbahn der Hin- und Herbewegung passiert, die Ultraschallenergie auf einen dem zweiten Abschnitt des flächigen Elements entsprechenden zweiten Teil aufgebracht wird, um den zweiten Teil zu schweißen, und
wenn das Ultraschall-Verarbeitungselement den ersten Abschnitt (A1ab) auf einer Rückkehrbahn der Hin- und Herbewegung passiert, die Ultraschallenergie auf einen dem ersten Abschnitt des flächigen Elements entsprechenden ersten Teil aufgebracht wird, um den ersten Teil zu schweißen.

7. Ultraschallenergie-Schweißvorrichtung eines mit einem saugfähigen Artikel assoziierten flächigen Elements nach Anspruch 1 oder 2, wobei
ein Pressmechanismus enthalten ist, wobei der Pressmechanismus ein Ultraschall-Verarbeitungselement von dem Horn (61) und dem Amboss (71) mit einer Presskraft an ein anderes Ultraschall-Verarbeitungselement presst, um das flächige Element mit dem Horn und dem Amboss zu klemmen, und
die Ultraschallenergie-Reguliereinheit bewirkt, dass sich die Menge (J/m) der pro Längeneinheit aufgebrachten Ultraschallenergie ändert, indem sie bewirkt, dass sich der Betrag der Presskraft ändert.

8. Ultraschallenergie-Schweißvorrichtung eines mit einem saugfähigen Artikel assoziierten flächigen Elements nach Anspruch 1 oder 2, wobei
die Ultraschallenergie-Reguliereinheit bewirkt, dass sich eine Menge (J/m) der pro Längeneinheit aufgebrachten Ultraschallenergie ändert, ändert, indem sie bewirkt, dass sich eine Größe eines Zwischenraums (G) zwischen dem Horn (61) und dem Amboss (71) ändert.

9. Ultraschallenergie-Schweißvorrichtung eines mit einem saugfähigen Artikel assoziierten flächigen Elements nach Anspruch 1 oder 2, wobei
die Ultraschallenergie-Reguliereinheit bewirkt, dass sich eine Menge (J/m) der pro Längeneinheit aufgebrachten Ultraschallenergie (J/m) ändert, indem sie bewirkt, dass sich ein Verfahrgeschwindigkeitswert, mit dem sich das Ultraschall-Verarbeitungselement in der CD-Richtung bewegt, ändert.

10. Ultraschall-Schweißvorrichtung eines mit einem saugfähigen Artikel assoziierten flächigen Elements nach einem der Ansprüche 1 bis 9, wobei
das Ultraschall-Verarbeitungselement, bei dem es sich um eines von dem Horn (61) und dem Amboss (71) handelt, ein Walzenelement umfasst,
das Walzenelement drehbar vorgesehen ist und in Bezug auf die Außenumfangsfläche (30s) des rotierenden Elements außen angeordnet ist, und
sich das Walzenelement in der CD-Richtung bewegt, während es auf einem schienenartigen Element rollt,
das sich in der CD-Richtung erstreckend vorgesehene schienenartige Element sich relativ zu der Außenumfangsfläche des rotierenden Elements nicht bewegen darf,
das schienenartige Element als ein weiteres Ultraschall-Verarbeitungselement dient.

11. Ultraschall-Schweißvorrichtung eines mit einem saugfähigen Artikel assoziierten flächigen Elements nach einem der Ansprüche 1 bis 9, wobei
sich sowohl das Horn (61) als auch der Amboss (71) in der CD-Richtung hin- und herbewegen, während das flächige Element zwischen dem Horn und dem Amboss geklemmt wird.

12. Ultraschall-Schweißverfahren, bei dem ein mit einem saugfähigen Artikel assoziiertes flächiges Element (1a) Ultraschallschweißen unterzogen wird,
wobei das flächige Element transportiert wird, während es auf eine Außenumfangsfläche (30s) eines rotierenden Elements (30) gewickelt ist,
wobei das rotierende Element um seine Mittelachse (C30) rotiert,
wobei das Verfahren Folgendes umfasst:
Rotieren einer Ultraschall-Verarbeitungseinheit (60) um die Mittelachse (C30) zusammen mit dem rotierenden Element (30),
wobei die Ultraschall-Verarbeitungseinheit als Ultraschall-Verarbeitungselemente Folgendes umfasst:
ein Horn (61), und
einen dem Horn gegenüberliegenden Amboss (71);
Bewirken, dass das Horn mit Ultraschallfrequenz schwingt;
Bewirken, dass sich mindestens ein Ultraschall-Verarbeitungselement von dem Horn und dem Amboss in einer CD-Richtung in Bezug auf einen Teil (1ap) des flächigen Elements hin- und herbewegt, wobei das Teil um das rotierende Element gewickelt ist, wobei die Hin- und Herbewegung ausgeführt wird, während das flächige Element zwischen dem Horn und dem Amboss geklemmt wird und wobei die CD-Richtung eine Transportrichtung des flächigen Elements kreuzt,
wobei sich beim Hin- und Herbewegen das mindestens eine Ultraschall-Verarbeitungselement von dem Horn und dem Amboss zu einer Überquerungsposition (Pf, Pb) bewegt, die in der CD-Richtung hinter einem Endteil (1aef, 1aeb) des flächigen Elements liegt, wobei das Horn und der Amboss in der Überquerungsposition einander gegenüberliegen,
wobei die Außenumfangsfläche (30s) des rotierenden Elements Abschnitte umfasst, in denen jeder Endteil des flächigen Elements zu positionieren ist,
**dadurch gekennzeichnet, dass** im Fall, dass es sich von den Abschnitten bei einem Abschnitt, den das mindestens eine Ultraschall-Verarbeitungselement von dem Horn und dem Amboss beim Beginn der Überquerung des flächigen Elements in der CD-Richtung passiert, um einen ersten Abschnitt (A1aeb) handelt, und es sich bei einem Abschnitt, den das mindestens eine Ultraschall-Verarbeitungselement beim Abschließen der Überquerung passiert, um einen zweiten Abschnitt (A1aef) handelt,
Bewirken, dass eine Menge (J/m) der pro Längeneinheit in der CD-Richtung aufzubringenden Ultraschallenergie beginnt, abzunehmen, wenn das Ultraschall-Verarbeitungselement den zweiten Abschnitt (A1aef) passiert.

## Revendications

1. Appareil de soudage ultrasonique (20) qui effectue un soudage ultrasonique sur un élément en forme de feuille (1a), associé à un article absorbant,
cet élément en forme de feuille étant transporté tout en étant enroulé sur une face circonférentielle extérieure (30s) d'un élément rotatif (30),
cet élément rotatif tournant autour de son axe central (C30), cet appareil comprenant :
l'élément rotatif (30) ; et
une unité de traitement ultrasonique (60)
qui tourne autour de l'axe central (C30) avec l'élément rotatif, et
qui comprend, comme éléments de traitement ultrasonique,
un pavillon (61) qui vibre ultrasoniquement et
une enclume (71) qui prend en sandwich l'élément en forme de feuille avec le pavillon ;
au moins l'un ou l'autre des éléments de traitement ultrasonique du pavillon (61) et de l'enclume (71) étant guidé de manière à être capable d'effectuer un mouvement de va-et-vient dans une direction CD, cette direction CD coupant une direction de transport de l'élément en forme de feuille,
au moins l'un ou l'autre des éléments de traitement ultrasonique du pavillon et de l'enclume effectuant un mouvement de va-et-vient dans la direction CD par rapport à une partie (1ap) de l'élément en forme de feuille, cette partie étant enroulée autour de l'élément rotatif,
dans le mouvement de va-et-vient, au moins l'un ou l'autre élément de traitement ultrasonique du pavillon et de l'enclume bougeant jusqu'à une position de croisement effectué (Pf, Pb) qui est située au-delà d'une partie extrême (1aef, 1aeb) de l'élément en forme de feuille dans la direction CD, le pavillon et l'enclume se faisant face l'un à l'autre dans la position de croisement effectué,
la face circonférentielle extérieure (30s) de l'élément rotatif comprenant des sections dans lesquelles chaque partie extrême de l'élément en forme de feuille doit être positionnée,
**caractérisé en ce que** cet appareil comprend en outre une unité de régulation de l'énergie ultrasonique, et
dans le cas où, parmi les sections, une section devant laquelle au moins l'un ou l'autre élément de traitement ultrasonique du pavillon et de l'enclume passe lorsqu'il commence à croiser l'élément en forme de feuille dans la direction CD étant une première section (A1aeb), une section devant laquelle au moins l'un ou l'autre élément de traitement ultrasonique du pavillon et de l'enclume passe lorsqu'il finit le croisement étant une deuxième section (A1aef),
l'unité de régulation de l'énergie ultrasonique faisant en sorte qu'une quantité (J/m) d'énergie ultrasonique à appliquer par unité de longueur dans la direction CD commence à diminuer, lorsque l'élément de traitement ultrasonique passe devant la deuxième section (A1aef).

2. Appareil de soudage ultrasonique d'un élément en forme de feuille associé à un article absorbant selon la revendication 1, dans lequel
l'élément de traitement ultrasonique, après être passé jusqu'à une section extérieure (Aef) positionnée à l'extérieur de la deuxième section (A1aef) dans la direction CD, fait demi-tour dans la position de croisement effectué comme une position de demi-tour,
lorsque l'élément de traitement ultrasonique passe devant la section extérieure avant de faire demi-tour dans la position de demi-tour, l'unité de régulation de l'énergie ultrasonique diminue encore la quantité (J/m) de l'énergie ultrasonique.

3. Appareil de soudage ultrasonique d'un élément en forme de feuille associé à un article absorbant selon la revendication 1 ou 2, dans lequel
l'unité de régulation de l'énergie ultrasonique fait changer la quantité (J/m) de l'énergie ultrasonique à appliquer par unité de longueur en faisant changer une amplitude de la vibration ultrasonique du pavillon (61).

4. Appareil de soudage ultrasonique d'un élément en forme de feuille associé à un article absorbant selon la revendication 3, dans lequel
un mécanisme de pression est compris, ce mécanisme de pression pressant avec une force de pression un élément de traitement ultrasonique du pavillon (61) et de l'enclume (71) sur un autre élément de traitement ultrasonique, de façon à prendre en sandwich l'élément en forme de feuille avec le pavillon et l'enclume, et
ce mécanisme de pression commence à diminuer la force de pression tandis que l'amplitude diminue.

5. Appareil de soudage ultrasonique d'un élément en forme de feuille associé à un article absorbant selon la revendication 3 ou 4, dans lequel un mécanisme de changement d'espace qui change un espace (G) entre le pavillon (61) et l'enclume (71) est compris, et
ce mécanisme de changement d'espace commence à augmenter l'espace tandis que l'amplitude diminue.

6. Appareil de soudage ultrasonique d'un l'élément en forme de feuille associé à un article absorbant selon l'une quelconque des revendications 3 à 5, dans lequel,
dans le cas où une section de l'élément en forme de feuille (1a) que l'élément de traitement ultrasonique traverse dans la face circonférentielle extérieure (30s) de l'élément rotatif est une section de croisement (A1a),
cette section de croisement comprend une première section (A1ab) devant laquelle l'élément de traitement ultrasonique passe en premier dans un trajet vers l'avant du mouvement de va-et-vient et une deuxième section (A1af) devant laquelle l'élément de traitement ultrasonique passe après la première section dans le trajet vers l'avant,
lorsque l'élément de traitement ultrasonique passe devant la deuxième section (A1af) dans le trajet vers l'avant du mouvement de va-et-vient, l'énergie ultrasonique est appliquée sur une deuxième partie correspondant à la deuxième section de l'élément en forme de feuille, pour souder cette deuxième partie, et
lorsque l'élément de traitement ultrasonique passe devant la première section (A1ab) dans un trajet de retour du mouvement de va-et-vient, l'énergie ultrasonique est appliquée sur une première partie correspondant à la première section de l'élément en forme de feuille, pour souder cette première partie.

7. Appareil de soudage ultrasonique d'un élément en forme de feuille associé à un article absorbant selon la revendication 1 ou 2, dans lequel
un mécanisme de pression est compris, ce mécanisme de pression pressant avec une force de pression un élément de traitement ultrasonique du pavillon (61) et de l'enclume (71) sur un autre élément de traitement ultrasonique, de façon à prendre en sandwich l'élément en forme de feuille avec le pavillon et l'enclume, et
l'unité de régulation de l'énergie ultrasonique fait changer la quantité (J/m) de l'énergie ultrasonique qui est appliquée par unité de longueur en faisant changer la quantité de la force de pression.

8. Appareil de soudage ultrasonique d'un élément en forme de feuille associé à un article absorbant selon la revendication 1 ou 2, dans lequel
l'unité de régulation de l'énergie ultrasonique fait changer une quantité (J/m) de l'énergie ultrasonique qui est appliquée par unité de longueur en faisant changer une taille d'un espace (G) entre le pavillon (61) et l'enclume (71).

9. Appareil de soudage ultrasonique d'un élément en forme de feuille associé à un article absorbant selon la revendication 1 ou 2, dans lequel
l'unité de régulation de l'énergie ultrasonique fait chanter une quantité (J/m) de l'énergie ultrasonique qui est appliquée par unité de longueur, en faisant changer une valeur de vitesse de déplacement à laquelle l'élément de traitement ultrasonique bouge dans la direction CD.

10. Appareil de soudage ultrasonique d'un élément en forme de feuille associé à un article absorbant selon l'une quelconque des revendications 1 à 9, dans lequel
l'élément de traitement ultrasonique qui est soit le pavillon (61), soit l'enclume (71) comprend un élément rouleau,
cet élément rouleau est prévu de manière rotative et est disposé à l'extérieur par rapport à la face circonférentielle extérieure (30s) de l'élément rotatif, et
cet élément rouleau bouge dans la direction CD tout en roulant sur un élément semblable à un rail,
cet élément semblable à un rail étant prévu comme s'étendant dans la direction CD de façon à ne pas bouger par rapport à la face circonférentielle de l'élément rotatif,
cet élément semblable à un rail servant comme un autre élément de traitement ultrasonique.

11. Appareil de soudage ultrasonique d'un élément en forme de feuille associé à un article absorbant selon l'une quelconque des revendications 1 à 9, dans lequel
le pavillon (61) et l'enclume (71) effectuent tous les deux un mouvement de va-et-vient dans la direction CD tandis que l'élément en forme de feuille est pris en sandwich entre le pavillon et l'enclume.

12. Procédé de soudage ultrasonique dans lequel un élément en forme de feuille (1a) associé à un article absorbant est soumis à un soudage ultrasonique,
cet élément en forme de feuille étant transporté tout en étant enroulé sur une face circonférentielle extérieure (30s) d'un élément rotatif (30),
cet élément rotatif tournant autour de son axe central (C30),
ce procédé comprenant :
la rotation d'une unité de traitement ultrasonique (60) autour de l'axe central (C30) avec l'élément rotatif (30),
cette unité de traitement ultrasonique comprenant comme éléments de traitement ultrasonique,
un pavillon (61) et
une enclume (71) faisant face au pavillon ;
faisant en sorte que le pavillon vibre ultrasoniquement ;
faisant en sorte qu'au moins l'un ou l'autre élément de traitement ultrasonique du pavillon et de l'enclume effectue un mouvement de va-et-vient dans la direction CD par rapport à une partie (1ap) de l'élément en forme de feuille, cette partie étant enroulée autour de l'élément rotatif, ce mouvement de va-et-vient étant effectué tandis que l'élément en forme de feuille est pris en sandwich entre le pavillon et l'enclume, et la direction CD coupant une direction de transport de l'élément en forme de feuille,
dans le mouvement de va-et-vient, au moins l'un ou l'autre élément de traitement ultrasonique du pavillon et de l'enclume bougeant jusqu'à une position de croisement effectué (Pf, Pb) qui est située au-delà d'une partie extrême (1aef, 1aeb) de l'élément en forme de feuille dans la direction CD, le pavillon et l'enclume se faisant face l'un à l'autre dans la position de croisement effectué,
la face circonférentielle extérieure (30s) de l'élément rotatif comprenant des sections dans lesquelles chaque partie extrême de l'élément en forme de feuille doit être positionnée,
**caractérisé en ce que**, dans le cas où, parmi les sections, une section devant laquelle au moins l'un ou l'autre élément de traitement ultrasonique du pavillon et de l'enclume passe lorsqu'il commence à croiser l'élément en forme de feuille dans la direction CD étant une première section (A1aeb), une section devant laquelle au moins l'un ou l'autre élément de traitement ultrasonique passe lorsqu'il finit le croisement étant une deuxième section (A1aef),
faisant en sorte qu'une quantité (J/m) d'énergie ultrasonique à appliquer par unité de longueur dans la direction CD commence à diminuer, lorsque l'élément de traitement ultrasonique passe devant la deuxième section (A1aef).
